# EUROPEAN PATENT SPECIFICATION

(11) **EP 2 103 301 B1**
(45) Date of publication and mention of the grant of the patent: **13.02.2013**
(21) Application number: 09250776.3
(22) Date of filing: 20.03.2009
(51) Int. Cl.: A61K 8/894, C08G 77/46, C08G 77/50

(54) **Polyglycerin-modified silicone and a cosmetic comprising the same**
Polyglycerinmodifiziertes Silikon und Kosmetikprodukt damit
Silicone à polyglycérine modifiée et produit cosmétique la comprenant

(30) Priority: 21.03.2008 JP 2008074312; 06.03.2009 JP 2009053404
(43) Date of publication of application: 23.09.2009
(73) Proprietor: Shin-Etsu Chemical Co., Ltd., Tokyo (JP)
(72) Inventor: Sakuta, Koji, Annaka-shi, Gunma-ken (JP); Akabane, Emi, Annaka-shi, Gunma-ken (JP)
(74) Representative: Hallybone, Huw George

(56) References cited:
- EP-A- 1 213 316
- EP-A- 1 489 128
- US-A- 4 431 789
- US-A1- 2004 091 439

## Description

### FIELD OF THE INVENTION

The present invention relates to a polyglycerin-modified silicone and a cosmetic comprising the same. The polyglycerin-modified silicone is soluble in water, emulsifies various kinds of unctuous agents, and disperses powder.

### BACKGROUND OF THE INVENTION

Silicon oils are widely used for cosmetics for its excellent properties such as refreshing feel to the touch and safeness. To prepare a homogeneous emulsion from a silicone oil, water, an unctuous agent such as hydrocarbon, and powder, a silicone surfactant is used. Exemplary silicone surfactants include a polyoxyalkylene-modified silicone, a polyoxyalkylene-modified silicone having a long-chain alkyl group, and polyhydric alcohol-modified silicone as described in JP 2003-146832A and US 2004/0091439A1.

The polyoxyalkylene-modified silicone is thickened when it is mixed with water to make a cosmetic tacky to the touch. The polyhydric alcohol-modified silicone, particularly a glycerin-modified silicone, does not cause such problem, so that it is incorporated in cosmetics where good feel to the touch are desired, for example, hair cosmetics, as described in JP 2004-231607A and JP 2005-97151A.

EP 1 489 128 A1 describes a branched polyglycerol-modified silicone, silicon atoms of which are connected to at least one branched polyglycerol chain having one or more branched glycerol groups via a connecting group, a method for producing the same, and a cosmetic containing the same.

However, a silicone modified with a long polyglycerin chain is generally difficult to prepare due to poor miscibility between starting materials, i.e., glycerin and a silicone. Further, some glycerin-modified silicones cause cloudiness when they are incorporated in cosmetics containing water.

### SUMMARY OF THE INVENTION

The present invention is to provide a glycerin-modified silicone which does not cause aforesaid thickening problem or cloudiness problem.

As a result of extensive studies, the present inventors have found that a silicone which has a polyglyceryl group bonded to a silicon atom via a polyoxyalkylene group has excellent properties which are different from either a conventional glycerin-modified silicone or a polyether-modified silicone. The present invention is a glycerin-modified silicone represented by the following formula (1):

R¹ₐR²_{b}R³_{c}SiO_{(4-a-b-c)/2} (1)

wherein R¹ is, independently, an unsubstituted or a partially halogen-substituted alkyl, aryl, aralkyl, aminoalkyl, or carboxyalkyl group, of 1 to 30 carbon atoms,
R² is, independently, an organosiloxyalkyl group represented by the following formula (3): 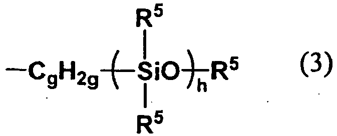 wherein R⁵ is an unsubstituted or a partially halogen-substituted alkyl group of 1 to 30 carbon atoms, g is a number of from 1 to 5, and h is a number of from 1 to 500,
R³ is a group represented by the following formula (4): 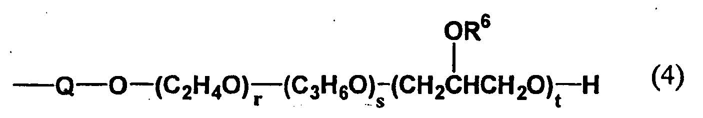 wherein Q is a C2-20 divalent group, r is a number of from 1 to 200, s is a number of from 0 to 200, R⁶ is a hydrogen atom, a C1-30 alkyl group or an organic group of the formula: -(CO)-R⁷, wherein R⁷ is a C1-30 hydrocarbon group, and t is a number of from 1 to 10,
a is a number of from 0.5 to 2.5, b is a number of from 0 to 1.5, and c is a number of from 0.001 to 1.5.

The aforesaid glycerin-modified silicone of the present invention is highly hydrophilic and is capable of forming translucent cosmetic. It emulsifies an unctuous agent and disperses powder very well to form a cosmetic which is stable with time, spreads smoothly on the skin, and gives non-tacky and moisturized feel to the touch.

### DETAILED DESCRIPTION OF THE PREFERRED EMBODIMENTS

The polyglycerin-modified silicone of the present invention is characterized in that it has R³ represented by the formula (4) shown below: 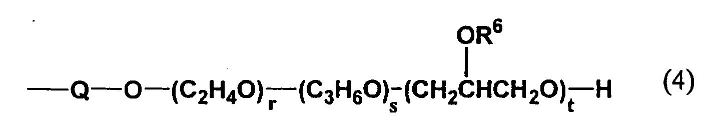 wherein a glycerin-origin group is bonded to a silicone main chain via a polyoxyalkylene group.

In the formula (4), r is a number of from 1 to 200, and s is a number of from 0 to 200. Preferably, r is a number of from 1 to 40 and s is a number of from 0 to 40. More preferably, r is a number of from 1 to 10, and s is a number of from 0 to 9. Most preferably, s is smaller than r, and particularly s is 0 and r ranges from 1 to 5. It should be noted that these numbers in the formulas (2), (3) and (4) are averaged numbers over a distributed molecular weight of the polymeric moieties such as (C₂H₄O)ᵣ.

R⁶ is a hydrogen atom, a C1-30 alkyl group or an organic group of the formula: -(CO)-R⁷, wherein R⁷ is a C1-30 hydrocarbon group, among which a hydrogen atom is most preferred. In the formula (4), t is a number of from 1 to 10, preferably from 1 to 5, and more preferably from 1 to 3.

In the formula (4), oxyethylene groups and oxypropylene groups are not necessarily bonded blockwise and can be bonded randomly. Further, either an oxyethylene group or an oxypropylene group can be bonded to the glycerin-origin group.

Q is a C2-20 divalent group. Examples of Q include -(CH₂)₂-, -(CH₂)₃-, -CH₂CH(CH₃)CH₂-, -(CH₂)₄-, -(C₂)₅-, -(CH₂)₆-, - (CH₂)₇-, -(CH₂)₈-, -(CH₂)₂-CH(CH₂CH₂CH₃)-, -CH₂ -CH (CH₂CH₃) -, and -CH₂-CH(CH₃)-COO(CH₂)₂-, among which -(CH₂)₂- -(CH₂)₃-, and -CH₂CH(CH₃)CH₂- are preferred, and -(CH₂)₃- is most preferred.

In the formula (1), each R¹ is, independently, an unsubstituted or a partially halogen-substituted alkyl, aryl, aralkyl, aminoalkyl, or carboxyalkyl group, of 1 to 30 carbon atoms. Examples of R¹ include alkyl groups such as methyl, ethyl, propyl, butyl, pentyl, hexyl, heptyl, octyl, nonyl, and decyl groups; cycloalkyl groups such as cyclopentyl and cyclohexyl groups; aryl groups such as phenyl and tollyl groups; aralkyl groups such as benzyl and phenetyl groups; amino-substituted alkyl groups such as 3-aminopropyl group, and 3-[(2-aminoethyl)amino]propyl groups; carboxyl-substituted alkyl groups such as 3-caroxypropyl group; and partially halogen-substituted alkyl groups mentioned above such as trifluoropropyl and nanofluorooctyl groups. Preferably, at least 50 %, more preferably at least 70%, of R¹ is a methyl group.

R¹ may comprise a polyoxyalkylene group represented by the following formula (2):

**-C_{d}H_{2d}-O-(C₂H₄O)ₑ -(C₃H₆O)_{f}-R⁴** (2)

in such an amount that the polyoxyalkylene group does not adversely affect the present invention. In the formula (2), R⁴ is a hydrogen atom, a partially halogen-substituted or unsubstituted C1-30 hydrocarbon group, or a C2-30 acyl group. Examples of R⁴ include the groups listed for the aforesaid R¹. In the formula (2), d is an integer of from 0 to 15, preferably from 3 to 5, or 11. The polyoxyalkylene group of the formula (2) with e = f = 0 represents an ally ether residue, pentenyl ether residue, or undecenyl ether residue. The polyoxyalkylene group of the formula (2) represents ally stearyl ether residue, pentenyl behenyl ether residue, or undecenyl oleyl ether residue when R⁴ is an alkyl group.

In the formula (2), each of e and f is, independently, a number of from 0 to 50, with e + f ranging from 1 to 200, and preferably from 2 to 30. When the polyoxyalkylene group of the formula (2) is composed of oxyethylene and oxypropylene groups, the both groups may constitute a block polymer or a random polymer.

In the formula (1), each R² is, independently, an organosiloxyalkyl group represented by the following formula (3) : 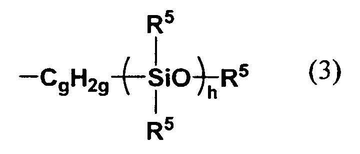 wherein h is a number of from 1 to 500, preferably from 3 to 100, and more preferably from 3 to 30. An organosiloxyalkyl group with h being larger than 500 is difficult to prepare. In the formula (3), g is a number of from 1 to 5, preferably 2 or 3, because such a group can be derived from a vinyl group or allyl group. R⁵ is a C1-30 alkyl group which may be partially halogen-substituted. Examples of R⁵ include those listed for the aforesaid R¹.

In the formula (1), a is a number of from 0.5 to 2.5, preferably from 1.0 to 2.3. If a is smaller than 0.5, miscibility with an unctuous agent is undesirably low. If a is larger than 2.5, desired hydrophilic property may not be achieved. In both cases, a stable oil-in-water type emulsion is difficult to prepare. In the formula (1), b is a number of from 0 to 1.5. If b is larger than 1.5, hydrophilicity is low, so that a stable oil-in-water type emulsion is difficult to prepare. In the formula (1), c is a number of from 0.001 to 1.5, preferably from 0.05 to 1.0. If c is smaller than 0.001, hydrophilicity is low, so that a stable oil-in-water type emulsion is difficult to prepare. If c is larger than 1.5, hydrophilicity is too high to prepare a stable oil-in-water type emulsion.

For use as an emulsifier, the silicone represented by the formula (1) preferably has a weight average molecular weight of from 500 to 20, 000, particularly from 1,000 to 10,000. For use in a skin cleansing composition, the silicone has a weight average molecular weight preferably of from 500 to 4, 000, more preferably 2,000 or smaller, and particularly 1,500 or smaller. For use as a powder dispersant, the silicone has a weight average molecular weight preferably of from 500 to 200, 000, more preferably from 1,000 to 100,000, and most preferably from 1,000 to 10,000.

The silicone of the present invention may have a unit represented by the following formula:

**R¹₁SiO_{3/2}**

to have a branch. In the above formula, R¹ is as defined above.

The silicone of the present invention can be prepared by addition- reacting SiH bonds of an organohydrogenpolysiloxane, which is to constitute a main chain of the silicone, with a compound represented by the formula (I) shown below having an unsaturated bond-containing group Q' bonded to an end of the group represented by the aforesaid formula (4).

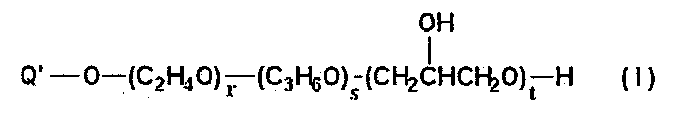

The silicone with R¹ being represented by the formula (2) can be prepared by addition-reacting the organohydrogenpolysiloxane with a compound represented by the following formula (III) having an unsaturated group and the polyoxyalkylene group.

**C_{d}H_{2d-1}-O-(C₂H₄O)₃-(C₃H₆O)_{f}-R⁴** (III)

The silicone with b being greater than 0 can be prepared by addition-reacting the organohydrogenpolysiloxane with a compound represented by the following formula (II) having an unsaturated group.

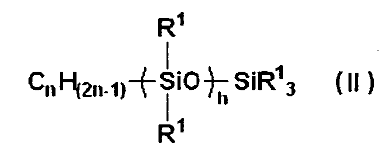

The silicone with R¹ being a long-chain alkyl group can be prepared by addition-reacting the organohydrogenpolysiloxane with an alkene compound corresponding to the long-chain alkyl group. The addition-reaction is performed in the presence of a platinum catalyst or rhodium catalyst.

In the above formulas (I) to (III), r, s, t, g, h, d, e, f, R¹ and R⁴ are as defined above. The organohydrogenpolysiloxane to constitute a main chain may be linear or cyclic, and preferably linear for the reason that the addition reaction progresses faster.

The compound of the above formula (I) can be prepared by reacting the hydroxyl group-containing compound represented by the following formula (IV): 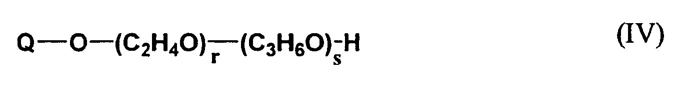 wherein an unsaturated group is present at an end of the group Q , with glycidol in the presence of an alkaline catalyst in an amount of 0.1 to 3 mol% per mole of the compound of the above formula (IV) at a temperature of from 100 °C to 130 °C.

The compounds of the aforesaid formulas (I) to (III), optionally the alkylene compound, are mixed with the organohydrogenpolysiloxane in such an amount that a molar ratio of a total unsaturated groups of the compounds per molar SiH bond of the organohydrogenpolysiloxane ranges from 0.5 to 2.0, preferably from 0.8 to 1.2.

As the platinum or rhodium catalyst used for the addition reaction, chloroplatinic acid, chloroplatinic acid modified with alcohol, or a chloroplatinic acid complex with a vinyl siloxane is preferably used. The catalyst can be used in an amount of a catalytically effective amount, and preferably at most 50 ppm, more preferably at most 20 ppm, as platinum metal or rhodium metal.

The addition reaction can be performed in an organic solvent. Examples of the organic solvent include aliphatic alcohols such as methanol, ethanol, 2-propanol, and butanol; aromatic hydrocarbons such as toluene and xylene; aliphatic or alicyclic hydrocarbons such as n-pentane, n-hexane, and cyclohexane; and halogenated hydrocarbons such as dichloromethane, chloroform, and carbon tetrachloride. The addition reaction is generally carried out under reflux of the organic solvent for 1 to 10 hours.

The present invention also provides a cosmetic comprising the aforesaid silicone of the present invention, which may be hereinafter referred to as the silicone (A). A content of the silicone (A) in the cosmetic may vary depending on an intended use of the silicone. When the silicone is used as an emulsifier, the content ranges from 0.01 to 20 mass%, preferably from 0.1 to 10 mass%, based on total mass of the cosmetic. When the silicone is used as a detergent, the content rages from 0.1 to 40 mass%, preferably from 2 to 30 mass%. When the silicone is used as a powder dispersant, the content ranges from 0.1 to 30 mass%, preferably from 0.5 to 10 mass %, based on total mass of powder to be dispersed.

Examples of the cosmetic include various kinds of externally used cosmetics, for example, makeup products such as lipstick, and oil foundation, and skin care products, hairdressing products, medicine for external application, face wash, and makeup remover.

The cosmetic of the present invention may comprise various components commonly incorporated in cosmetics such as (B)an unctuous agent, (C) a compound having an alcoholic hydroxyl group, (D)water, (E) a powder, (F) a colorant, (G) a surfactant, (H) a crosslinked organopolysiloxane, and (I)a silicone resin. The components will be explained successively.

The unctuous agent (B) may be solid, semi-solid or liquid. Examples of the unctuous agent include natural plant or animal oils and semi-synthetic oils, for example, avocado oil, linseed oil, almond oil, Ibota wax, perilla oil, olive oil, cacao butter, kapok wax, kaya oil, carnauba wax, Glycyrrhiza oil, candelilla wax, beef tallow, neat's-foot oil, beef bone fat, hydrogenated beef tallow, apricot kernel oil, spermaceti wax, hydrogenated oil, wheat germ oil, sesame oil, rice germ oil, rice bran oil, sugar cane wax, sasanqua oil, safflower oil, shear butter, Chinese tung oil, cinnamon oil, jojoba wax, shellac wax, turtle oil, soybean oil, tea seed oil, camellia oil, evening primrose oil, corn oil, lard, rapeseed oil, Japanese tung oil, rice bran oil, germ oil, horse fat, persic oil, palm oil, palm kernel oil, castor oil, hydrogenated castor oil, castor oil fatty acid methylester, sunflower oil, grape oil, bayberry wax, jojoba oil, macadamia nut oil, beeswax, mink oil, cottonseed oil, cotton wax, Japanese wax, Japanese wax kernel oil, montan wax, coconut oil, hydrogenated coconut oil, tri-coconut oil fatty acid glyceride, mutton tallow, peanut oil, lanolin, liquid lanolin, hydrogenated lanolin, lanolin alcohol, hard lanolin, lanolin acetate, isopropyl lanolate, hexyl laurate, POE lanolin alcohol ether, POE lanolin alcohol acetate, polyethylene glycol lanolate, POE hydrogenated lanolin alcohol ether, and egg yolk oil, wherein POE represents polyoxyethylene.

The unctuous agent (B) may be a hydrocarbon oil of which examples include ozokerite, squalane, squalene, ceresin, paraffin, paraffin wax, liquid paraffin, pristane, polyisobutylene, microcrystalline wax, vaseline and higher fatty acids, e.g., lauric acid, myristic acid, palmitic acid, stearic acid, behenic acid, undecylenic acid, oleic acid, linoleic acid, linolenic acid, arachidonic acid, eicosapentaenoic acid (EPA), docosahexaenoic acid (DHA), isostearic acid, and 12-hydroxystearic acid.

The unctuous agent (B) may be a higher alcohol of which examples of include lauryl alcohol, myristyl alcohol, palmityl alcohol, stearyl alcohol, behenyl alcohol, hexadecyl alcohol, oleyl alcohol, isostearyl alcohol, hexyldodecanol, octyl dodecanol, cetostearyl alcohol, 2-decyltetradecinol, cholesterol, phytosterol, POE cholesterol ether, monostearyl glycerin ether (batyl alcohol), and monooleyl glyceryl ether (cerakyl alcohol).

The unctuous agent (B) may be an ester oil of which examples include diisobutyl adipate, 2-hexyldecyl adipate, di-2-heptylundecyl adipate, N-alkyl glycol monoisostearate, isocetyl isostearate, trimethylolpropane triisostearate, ethylene glycol di-2-ethylhexanoate, cetyl 2-ethylhexanoate, trimethylolpropane tri-2-ethylhexanoate, pentaerythritol tetra-2-ethylhexanoate, cetyl octanoate, octyldodecyl gum ester, oleyl oleate, octyldodecyl oleate, decyl oleate, isononyl isononanate, neopentyl glycol dicaprirate, triethyl citrate, 2-ethylhexyl succinate, amyl acetate, ethyl acetate, butyl acetate, isocetyl stearate, butyl stearate, diisopropyl sebacinate, di-2-ethylhexyl sebacinate, cetyl lactate, myristyl lactate, isopropyl palmitate, 2-ethylhexyl palmitate, 2-hexyldecyl palmitate, 2-heptylundecyl palmitate, cholesteryl 12-hydroxystearate, dipentaerythritol fatty acid esters, isopropyl myristate, octyldodecyl myristate, 2-hexyldecyl myristate, myristyl myristate, hexyldecyl dimethyloctanoate, ethyl laurate, hexyl laurate, 2-octyldodecyl N-lauroyl-L-glutamate, and diisostearyl. Examples of glyceride oils include acetoglyceryl, glycerol triisooctanoate, glyceryl triisostearate, glyceryl triisopalmitate, glyceryl monostearate, glyceryl di-2-heptylundecanoate, glyceryl trimyristate, and diglyceryl myristyl isostearate.

The unctuous agent (B) may be a conventionally used silicone oil of which examples include linear or branched organopolysiloxanes such as dimethylpolysiloxane, methylphenylpolysiloxane, methylhydrogenpolysiloxane, copolymer of dimethylsiloxane and methylphenylsiloxane; cyclic siloxanes such as octamethylcyclotetrasiloxane, decamethylcyclopentasiloxane, dodecamethylcyclohexasiloxane, tetramethyl-tetrahydrogencyclotetrasiloxane; tetramethyltetraphenylcyclotetrasiloxane; silicone rubber such as gummy dimethylpolysiloxanes having high polymerization degrees, and gummy dimethylsiloxane/methylphenylsiloxane copolymer; cyclosiloxane solutions of silicone gum or rubber, trimethylsiloxysilicate, cyclosiloxane solutions of trimethylsiloxysilicate, higher alkoxy-modified silicones such as stearoxysilicone, higher fatty acid-modified silicones, alkyl-modified silicones, amino-modified silicones, fluorosilicone, and solutions of silicone resins.

The unctuous agent (B) may be a fluorine-containing oil of which examples include perfluoropolyether, perfluorodecalin, and perfluorooctane.

The unctuous agent may be incorporated in the cosmetics in an amount of from 1 to 99 mass % based on a total mass of the cosmetic.

Examples of the compound having an alcoholic hydroxyl group (C) include lower alcohols such as ethanol, and isopropanol; sugar alcohols such as sorbitol, and maltose; sterols such as cholesterol, sitosterol, phytosterol, and lanosterol; and polyalcohols such as butylene glycol, propylene glycol, dibutylene glycol, and pentylene glycol.

As water (D), purified water, distilled water, and deionized water can be used. A content of water in the cosmetic can be adjusted in the range of from 1 to 99 mass %, based on total mass of the cosmetic.

As the powder (E), any powder which is commonly used in cosmetics may be used, regardless of the shape such as spherical, rod-like, acicular, or flake; of particle size such as fume grade, fine grade or pigment grade; and of particle structure such as porous and non-porous. Examples of the powder include inorganic powder, organic powder, surface active metal salt powder, colored pigments, pearl pigments, metallic powder, and natural colorants.

Examples of the inorganic powder include inorganic powder include titanium dioxide, zirconium oxide, zinc oxide, cerium oxide, magnesium oxide, barium sulfate, calcium sulfate, magnesium sulfate, calcium carbonate, magnesium carbonate, talc, mica, kaolin, sericite, white mica, synthetic mica, phlogopite, lepidolite, biotite, lithia mica, silicic acid, silicic anhydride, aluminum silicate, magnesium silicate, aluminum magnesium silicate, calcium silicate, barium silicate, strontium silicate, metal salts of tungstenic acid, hydroxyapatite, vermiculite, higilite, bentonite, montmorillonite, hectolitre, zeolite, ceramics powder, calcium secondary phosphate, alumina, aluminum hydroxide, boron nitride, and silica.

Examples of the organic powder include polyamide powder, polyester powder, polyethylene powder, polypropylene powder, polystyrene powder, polyurethane powder, benzoguanamine powder, polymethylbenzoguanamine powder, tetrafluoroethylene powder, polymethylmethacrylate powder, cellulose powder, silk powder, powder of nylon such as Nylon 12 and Nylon 6, crosslinked dimethylsilicone elastomer powder as described in Japanese Patent Application Laid-Open No. 3-93834, polymethylsylsesquioxane spherical powder as described in Japanese Patent Application Laid-Open No. 3-47848, silicone elastomer spherical powder coated with polymethylsylsesquioxane as described in Japanese Patent Application Laid-Open No. 7-196815, powder of styrene / acrylic acid copolymer, divinylbenzene / styrene copolymer, vinyl resin, urea resin, phenol resin, fluororesin, silicone resin, acrylic resin, melamine resin, epoxy resin, polycarbonate resin, microcrystalline fiber, starch powder, and lauroyl lysine.

Powder which scatters ultraviolet light can be used such as fine powder of titanium dioxide, iron-containing titanium dioxide, zinc oxide, cerium oxide and composite thereof.

The powder may be in the form of composite or may be treated with silicone oil, fluorine compound, or surfactant to the extent not to adversely affect the present cosmetics. For example, the powder may be treated in advance with oil, an amino acid, silicone oil, a fluorine compound or a surfactant, a linear or branched organopolysiloxane having hydrolysable silyl group or SiH bond, a linear or branched organopolysiloxane having long alkyl chain and hydrolysable silyl group or SiH bond, a linear or branched organopolysiloxane having polyoxyalkylene moiety and hydrolysable silyl group or SiH bond, an acryl silicone copolymer having hydrolysable silyl group or SiH bond. Two or more of the treatment agents may be employed. Commercially available powder treatment agents can be used, for example, AES-3083, KF-99P, KF-9901, KF-9908, KF-9909, and KP-574, all manufactured by Shin-Etsu Chemical Co., Ltd.

Examples of the colorants (F) include pigments and dyes. Examples of the colored pigments include inorganic red pigments such as iron oxide, iron hydroxide, and iron titanate, inorganic brown pigments such as γ- iron oxide, inorganic yellow pigments such as iron oxide yellow and loess, inorganic black pigments such as iron oxide black and carbon black, inorganic violet pigments such as manganese violet and cobalt violet, inorganic green pigments such as chromium hydroxide, chromium oxide, cobalt oxide, and cobalt titanate, inorganic blue pigments such as Prussian blue and ultramarine blue, lakes of tar pigments, lakes of natural dyes, and composite of these powder with a synthetic resin powder.

Examples of the pearl pigments include titanium dioxide-coated mica, bismuth oxychloride, titanium dioxide-coated bismuth oxychloride, titanium dioxide-coated talc, silica-coated mica titanium oxide, fish scales, and titanium dioxide-coated colored mica.

Examples of metallic powder include aluminum powder, copper powder and stainless steel powder.

Examples of the tar pigments include Red No. 3, Red No. 104, Red No. 106, Red No. 201, Red No. 202, Red No. 204, Red No. 205, Red No. 220, Red No. 226, Red No. 227, Red No. 228, Red No. 230, Red No. 401, Red No. 505, Yellow No. 4, Yellow No. 5, Yellow No. 202, Yellow No. 203, Yellow No. 204, Yellow No. 401, Blue No. 1, Blue No. 2, Blue No. 201, Blue No. 404, Green No. 3, Green No. 201, Green No. 204, Green No. 205, Orange No. 201, Orange No. 203, Orange No. 204, Orange No. 206, and Orange No. 207; and natural pigments such as carminic acid, laccaic acid, carthamin, brazilin, and crocin.

The powder or colorant can be incorporated in the present cosmetic in an amount of from 0 to 99 mass % based on total mass of the cosmetic. Particularly in the powder cosmetic, the powder is preferably incorporated in an amount of from 80 to 90 mass% of the cosmetic.

As the surfactant (G), anionic, cationic, nonionic or amphoteric surfactant can be used. Examples of the anionic surfactant include fatty acid soaps, such as sodium stearate and triethanolamine palmitate, alkylether carboxylic acids and salts thereof, salts of condensates of amino acids with fatty acids, alkyl sulfonate salts, alkenesulfonates, sulfonates of fatty acid esters, fatty acid amide sulfonates, sulfonate salts of the formalin condensates, salts of alkyl sulfates, salts of secondary higher alcohol sulfates, salts of alkyl/allyl ether sulfates, salts of fatty acid ester sulfates, salts of fatty acid alkylolamide sulfates, and salts of Turkey Red oil sulfate, alkyl phosphate salts, ether phosphate salts, alkylallylether phosphate salts, amide phosphate salts, and N-acylamino surfactants.

Examples of the cationic surfactants include amine salts such as alkylamine salts, amine salts of polyamine and amino alcohol fatty acid derivatives, alkyl quaternary ammonium salts, aromatic quaternary ammonium salts, pyridinium salts and imidazolium salts.

Examples of the nonionic surfactants include sorbitan fatty acid esters, glycerin fatty acid esters, polyglycerin fatty acid esters, propylene glycol fatty acid esters, polyethylene glycol fatty acid esters, sucrose fatty acid esters, polyoxyethylene alkyl ethers, polyoxypropylene alkyl ethers, polyoxyethylene alkyl phenyl ether, polyoxyethylene fatty acid esters, polyoxyethylene sorbitan fatty acid esters, polyoxyethylene sorbitol fatty acid esters, polyoxyethylene glycerin fatty acid esters, polyoxyethylene propylene glycol fatty acid esters, polyoxyethylene castor oil, polyoxyethylene hydrogenated castor oil, polyoxyethylene phytostanol ether, polyoxyethylene phytosterol ether, polyoxyethylene cholestanol ether, polyoxyethylene cholesteryl ether, polyoxyalkylene-modified organopolysiloxane as described in JP No.2137062B, and JP No.7-330907A, polyoxyalkylene/alkyl-comodified organopolysiloxane as described in JP 61-90732A, JP 9-59386A, polyoxyalkylene- modified branched organopolysiloxane and polyoxyalkylene/alkyl-comodified branched organopolysiloxaneas described in JP2001-055307A, polyglycerin-modified organopolysiloxane as described in JP No.62-34039B, JP No.2613124B JP No.2844453B, and JP No.2002-179798, polyglycerin-modified branched-organopolysiloxane and polyglycerin/alkyl-comodified branched-organopolysiloxane described in JP 2002-179798A, alkanolamide, sugar ethers, and sugar amides. Examples of the amphoteric surfactant include betaine, aminocarboxylates, imidazoline derivatives, and amide amine type.

Among these surfactants, linear or branched organopolysiloxane having a polyoxyethylene moiety, linear or branched organopolysiloxane having polyoxyethylene and alkyl moieties, linear or branched organopolysiloxane having a polyglycerin moiety, and linear or branched organopolysiloxane having polyglycerin and alkyl moieties. These are sold under the trade names of KF-6011, KF-6012, KF-6013, KF-6015, KF-6016, KF-6017, KF-6028, KF-6028P, KF-6038, KF-6100, KF-6104, and KF-6105, all manufactured by Shin-Etsu Chemical Co., Ltd. Preferably, a surfactant having a HLB of from 1 to 10. A content of the surfactant in the cosmetic ranges preferably from 0.1 to 20 mass%, more preferably from 0.2 to 10 mass%, based on total mass of the cosmetic.

The cosmetic of the present invention may comprise one or more of crosslinked organopolysiloxanes (H), depending on a purpose of the cosmetic.

The crosslinked organopolysiloxane (H) preferably is swollen with a liquid unctuous agent in an amount larger than a weight of the organopolysiloxane (H). Examples of the liquid unctuous agents include silicone oil such as silicone oil having a viscosity of from 0.65 to 100.0 mm²/sec (25°C); hydrocarbon oil such as liquid paraffin, squalane, isododecane; glyceride oil such as triisooctanoic acid glycride and isotridecyl isononanate; ester oil such as N-amyl glutamate and lauroyl sarcosine; and natural animal or plant oil such as macadamia nuts oil.

Preferred crosslinked organopolysiloxane is a reaction product of an organopolysiloxane having at least two alkenyl groups per molecule and an organohydrogenpolysiloxane having a Si-H bond. Examples of organopolysiloxane having at least two alkenyl groups per molecule include an organopolysiloxane having at least two vinyl groups, a polyoxyalkylene having at least two ally groups, a polyglycerin having at least two ally groups, and α,ω-alkenyldiene. The crosslinked organopolysiloxane may have at least one group selected from polyoxyalkylene, polygryceryl, alkyl, alkenyl, aryl and fluoroalkyl groups. The crosslinked organopolysiloxanes are commercially available under the trade names of KSG-15, KSG-16, KSG-18, KSG-1610, USG-103, KSG-210, KSG-240, KSG-710, which are in the form of paste in a silicone oil, USG-106, KSG-41, KSG-42, KSG-43, KSG-44, KSG-310, KSG-320, KSG-330, KSG-340, KSG-810, KSG-820, KSG-830, KSG-840, which as in the form of paste in a triglyceride oil or hydrocarbon oil, all from Shin-Etsu Chemical Co., Ltd.

The crosslinked organopolysiloxanes may be incorporated in the cosmetic preferably in an amount of from 0.01 to 40 mass%, more preferably from 0.1 to 30 mass %, based on a total weight of the cosmetic.

The present cosmetic may further comprise a conventional silicone resin (I). Preferred silicone resins are acrylic silicones such as an acryl/silicone graft copolymer and acryl/silicone block copolymer. Preferably, use may be made of an acryl/silicone resin having at least one group selected from the group consisting of pyrrolidonyl, long-chain alkyl, polyoxyalkylene, and fluoroalkyl groups and anionic groups such as a carboxyl group. Such silicone resins are commercially available in the form of solutions in a silicone oil, hydrocarbon oil, or alcohol, under the trade names of KP-541, KP-543, KP-545, KP-549, KP-550, KP-571, KP-575, and KP-581, all from Shin-Etsu Chemical Co., Ltd.

Preferably, the silicone resin is a silicone network resin represented as MQ, MDQ, MT, MDT, or MDTQ, wherein M stands for R₃SiO_{0.5}, D for R₂ SiO, T for RSiO_{1.5}, and Q for SiO₂, widely used in the silicone industry. Generally, the silicone network resin is known as MQ resin, MT resin or MDT resin, which may have structures represented as MDQ, MTQ, and MDTQ. The silicone resin dissolved in decamethylcyclopentasiloxane or dimethicone is commercially available. The silicone network compound may have at least one moiety selected from the group consisting of pyrrolidone, long-chain alkyl, polyoxyalkylene, fluoroalkyl, and amino moieties.

The acrylic silicone resin and/or the silicone network resin can be incorporated in the cosmetics in an amount preferably of from 0.1 to 20 mass%, more preferably from 1 to 10 mass%, based on total weight of the cosmetic.

The present cosmetic may comprise a silicone wax (J) according to an intended use of the cosmetic. Preferred silicone wax is a polysiloxane modified with a polylactone that is a polymer obtained by ring-opening polymerization of a lactone compound having an at least five-membered ring.

Another preferred silicone wax is an acryl-modified silicone having at least one group selected from the group consisting of pyrrolidonyl, long-chain alkyl, polyoxyalkylene, fluoroalkyl, and anionic groups such as a carboxyl group. Silicone waxes having a long-chain alkyl group are sold under the trade names of KP-561P and KP-562P, both from Shin-Etsu Chemical Co., Ltd.

Still another preferred silicone wax is a silicone-modified olefin wax produced by addition-reacting an olefin wax with an organohydrogenpolysiloxane having at least one SiH bond per molecule. The olefin wax is a copolymer of ethylene with at least one diene, or a copolymer of ethylene, at least one C3-12 α -olefin, and at least one diene, preferably vinylnorbornene.

The silicone wax is incorporated in the cosmetic in an amount preferably of from 0.1 to 20 mass%, more preferably of from 1 to 10 mass %, based on total mass of the cosmetic.

In the cosmetic of the present invention, other components that are commonly used in cosmetics can be incorporated in an amount not to adversely affect the cosmetic. Examples of the components include oil-soluble gelling agents, clay minerals modified with organic compounds, resins, antiperspirants, ultraviolet absorbents, ultraviolet absorbing and scattering agents, moisture retention agents, antiseptics, anti-microbial agents, perfumes, salts, antioxidants, pH regulators, a chelating agents, refreshing agents, an anti-inflammatory agent, skin beautifying components, such as skin whitener, cell activator, rough dry skin improver, blood circulation promoter, skin astringent and anti-seborrheic agent, vitamins, amino acids, nucleic acids, hormones, clathrate compounds, and hair setting agents.

Examples of the oil-soluble gelling agent include metal soaps, such as aluminum stearate, magnesium stearate and zinc myristate; amino acid derivatives, such as N-lauroyl-L-glutamic acid and α, γ -di-n-butylamine; dextrin fatty acid esters, such as dextrin palmitic acid ester, dextrin stearic acid ester and dextrin 2-ethylhexaminic acid palmitic acid ester; inulin fatty acid esters such as fructooligostearate; sucrose fatty acid esters, such as sucrose palmitic acid ester and sucrose stearic acid ester; benzylidene derivatives of sorbitol, such as monobenzylidene sorbitol and dibenzylidene sorbitol; and clay minerals modified with organic compounds, such as dimethylbenzyldodecyl ammonium montmorillonite clay and dimethyldioctadecyl ammonium montmorillonite clay.

Examples of the antiperspirant include aluminum chlorohydrate, aluminum chloride, aluminum sesquichlorohydrate, zirconium hydoxychloride, aluminum zirconium hydroxychloride, and aluminum zirconium glycine complex.

Examples of the UV absorbents include UV absorbents of benzoic acid type such as p-aminobenzoic acid; those of anthranilic acid type such as methyl anthranilate; those of salicylic acid type such as methyl salicylate, octyl salicylate, and trimethylcyclohexyl salicylate; those of succinic acid type such as octyl p-methoxysuccinate; those of benzophenone type, such as 2,4-dihydroxybenzophenone; those of urocanic acid type, such as ethyl urocanate; those of dibenzoylmethane type such as 4-t-butyl-4'-methoxydibenzoylmethane; and phneylbenzoimidazol sulfonic acid. Examples of the ultraviolet scattering agents include fine powder of titanium oxide, fine powder of iron-containing titanium oxide, fine powder of zinc oxide, fine powder of cerium oxide, and a mixture of these powders. Dispersion of these ultraviolet absorbing or scattering agents in an oil can be used, too.

Examples of moisture retention agents include glycerin, sorbitol, propylene glycol, dipropylene glycol, 1,3-butylene glycol, pentylene glycol, glucose, xylitol, maltitol, polyethylene glycol, hyaluronic acid, chondroitin sulfuric acid, pyrrolidone carboxylate, polyoxyethylene glycoside, and polyoxypropylene methylglycoside.

Examples of the antiseptics include alkyl paraoxybenzoates, benzoic acid, sodium benzoate, sorbic acid, potassium sorbate, and phenoxyethanol may be used. For the antibacterial agents, benzoic acid, salicylic acid, carbolic acid, sorbic acid, paraoxybenzoic acid alkyl esters, parachloromethacresol, hexachlorophene, benzalkonium chloride, chlorohexydine chloride, trichlorocarbanilide and phenoxyethanol.

Examples of the salts include inorganic salts, organic acid salts, salts of amine and salts of amino acids. Examples of the inorganic salts include sodium, potassium, magnesium, calcium, aluminum, zirconium, or zinc salt of inorganic acid such as hydrochloric acid, sulfuric acid, carbonate acid, and nitric acid. Examples of the salts of organic acid include salts of organic acid such as acetic acid, dehydroacetic acid, citric acid, malic acid, succinic acid, ascorbic acid, and stearic acid. Examples of the salts of amine or amino acid include salt of triethanol amine and salt of glutamic acid. Other examples are salt of hyaluronic acid, chondroitin sulfate, aluminum/zirconium/glycine chelate, and salts produced by acid-alkaline neutralization reaction in the cosmetic.

Examples of the antioxidants include tocopherol, butylhydroxyanisole, dibutylhydroxytoluene and phytic acid; examples of the pH regulators include lactic acid, citric acid, glycolic acid, succinic acid, tartaric acid, dl-malic acid, potassium carbonate, sodium hydrogen carbonate and ammonium hydrogen carbonate; examples of the chelating agents include alanine, sodium ethylenediamine tetraacetate, sodium polyphosphate, sodium metaphosphate and phosphoric acid; examples of the refrigerants include L-menthol and camphor; and examples of the anti-inflammatory agents include allantoin, glycyrrhizin and salts thereof, glycyrrhetinic acid and stearyl glycyrrhetinate, tranexamic acid and azulene.

Examples of the skin-beautifying components include whitening agents, such as placenta extract, arbutin, glutathione and Yukinoshita extract; cell activators, such as royal jelly, photosensitizers, cholesterol derivatives and calf blood extract; rough and dry skin improvers; blood circulation improvers, such as nonylic acid vanillyl amide, benzyl nicotinate, beta -butoxyethyl nicotinate, capsaicin, zingerone, cantharis tincture, ichtammol, caffeine, tannic acid, alpha-borneol, tocopheryl nicotinate, inositol hexanicotinate, cyclandelate, cinnarizine, tolazoline, acetyl choline, verapamil, cepharanthin and gamma -oryzanol; skin astringents, such as zinc oxide and tannic acid; and anti-seborrheic agents, such as sulfur and thianthol.

Examples of the vitamins include vitamin A, such as vitamin A oil, retinol, retinyl acetate and retinyl palmitate; vitamin B, including vitamin B₂ such as riboflavin, riboflavin butyrate and flavin adenine nucleotide, vitamin B₆ such as pyridoxine hydrochloride, pyridoxine dioctanoate and pyridoxine tripalmitate, vitamin B₁₂ and its derivatives, and vitamin B15 and its derivatives; vitamin C, such as L-ascorbic acid, L-ascorbic acid dipalmitic ester, sodium (L-ascorbic acid)-2-sulfate and dipotassium L-ascorbic acid diphosphate; vitamin D, such as ergocalciferol and cholecarciferol; vitamin E, such as alpha -tocopherol, beta -tocopherol, gamma -tocopherol, dl- alpha -tocopheryl acetate, dl- alpha -tocopheryl nicotinate and dl- alpha -tocopheryl succinate; vitamin H; vitamin P; nicotinic acids, such as nicotinic acid, benzyl nicotinate and nicotinic acid amide; pantothenic acids, such as calcium pantothenate, D-pantothenyl alcohol, pantothenyl ethyl ether and acetylpantothenyl ethyl ether; and biotin.

Examples of the amino acids include glycine, valine, leucine, isoleucine, serine, threonine, phenylaranine, alginine, lysine, aspartic acid, glutamic acid, cystine, cysteine, methionine, and tryptophan; examples of the nucleic acids include deoxyribonucleic acid; and examples of the hormones include estradiol and ethenyl estradiol.

Examples of the polymers for hair setting include amphoteric, anionic, cationic, and nonionic polymers, such as polymers of polyvinyl pyrrolidone type such as polyvinyl pyrrolidone, vinyl pyrrolidone /vinyl acetate copolymers; acidic polymers of vinyl acetate ether type such as methyl vinyl ether / maleic acid anhydride alkyl half ester copolymer; polymers of acidic poly vinyl acetate type such as vinyl acetate / crotonic acid copolymer; acidic acrylic polymers such as (meth)acrylic acid / alkyl (meth) acrylate copolymer, (meth)acrylic acid / alkyl (meth) acrylate / alkyl acrylic amide copolymer, and amphoteric acrylic polymer such as N-methacryloylethyl-N,N-dimethylammonium alpha-N-methylcarboxybetaine / alkylmetahcrylate copolymer, hydroxypropyl (meth)acrylate/butylaminoethyl methacrylate / octyl amide of acrylic acid copolymer. Use is also made of naturally occurring polymers such as cellulose or derivatives thereof, keratin, collagen and derivatives thereof. Synthetic polymers can be used such as alkyd resin, epoxy resin, phenolic resin, unsaturated polyester resin, acryl copolymers, urethane resins, rubbers such NBR, SBR, SIS, SBS, and EPDM, and chlorinated polyethylene.

The cosmetic can be in various forms, for example, aqueous, oily, oil-in-water type emulsion, water-in-oil type emulsion, non-aqueous emulsion, and multi-emulsion such as W/O/W or O/W/O emulsion. Examples of the cosmetic include skin care cosmetic, such as face lotion, milky lotion, cream, face cleansing cream, massage materials, toilet soap and detergent, antiperspirant and deodorant; makeup cosmetic, such as face powder, foundation, rouge, eye shadow, mascara, eyeliner and lipstick; and hairdressing cosmetic, such as shampoo, rinse, treatment setting agent, antiperspirant and UV protective cosmetics, such as sunscreen milky lotion or sunscreen cream.

The present cosmetic may be in the various product forms such as lotion, milky lotion, cream, paste, gel, mousse, and spray.

The present cosmetic can be applied to the skin, nails, eyebrows, or eyelashes by a finger or an applicator such as brush, chip, a moldboard, and puff.

### EXAMPLES

The present invention is explained in further detail below with reference to examples, but the present invention is in no way limited by the examples. In the following, "%" means "% by mass" unless otherwise specified. Examples numbered with Roman figures are examples of the silicone of the present invention, and Examples numbered with Arabic figures are examples of the cosmetic composition of the present invention.

### Example I

In a reactor, 25 g of organohydrogenpolysiloxane represented by the following formula (5): 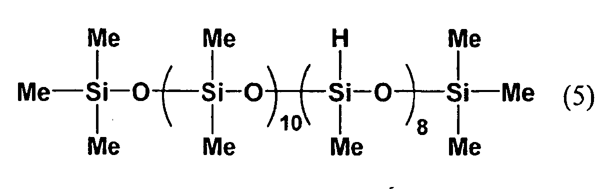 82 g of polyoxyalkylene group-containing compound represented by the following formula (6): 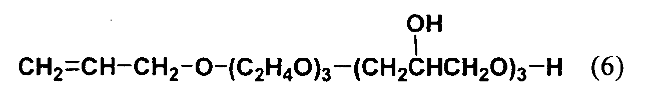 and 64 g of isopropyl alcohol were mixed, to which 0.03 g of 3% solution of chloroplatinic acid in ethanol was added. The mixture was then subjected to a reaction under reflux for 8 hours. Subsequently, the reaction mixture was vacuum-distilled and then filtered, whereby a silicone represented by the formula (7) shown below was obtained. The silicone was brown and transparent liquid which had a viscosity of 63000 mm²/s at 25 °C and a refractive index of 1.470 at 25 °C.

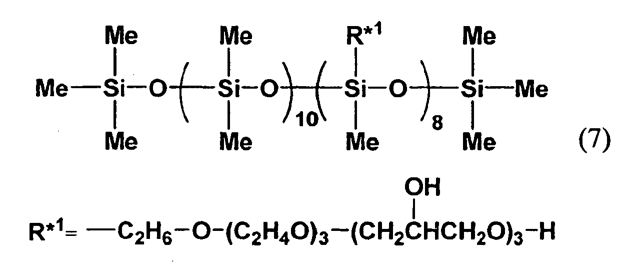

### Example II

In a reactor, 160 g of organohydrogenpolysiloxane represented by the following formula (8): 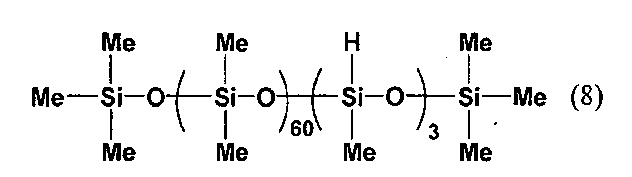 51 g of the aforesaid polyoxyalkylene group-containing compound of the average compositional formula (6), and 140 g of ethanol were mixed, to which 0.04 g of 3% solution of chloroplatinic acid in ethanol was added. The mixture was then subjected to a reaction under reflux for 8 hours. Subsequently, the reaction mixture was vacuum distilled and then filtered, whereby a silicone represented by the formula (9) shown below was obtained. The silicone was light brown and transparent liquid which had a viscosity of 960,000mm²/s at 25 °C and a refractive index of 1.422 at 25 °C. 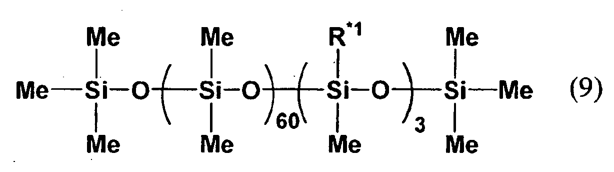 In the formula (9), R*¹ is as defined above.

### Example III

In a reactor, 338 g of organohydrogenpolysiloxane represented by the following formula (10): 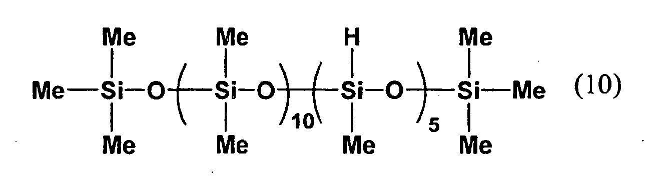 995 g of polyoxyalkylene group-containing compound represented by the following formula (11): 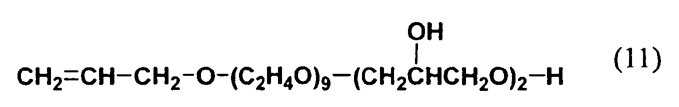 and 402 g of isopropyl alcohol were mixed, to which 0.2 g of 3% solution of chloroplatinic acid in ethanol was added. The mixture was then subjected to a reaction under reflux for 8 hours. Subsequently, the reaction solution was vacuum distilled, whereby a silicone represented by the following formula (12) was obtained. The silicone was light brown and transparent liquid which had a viscosity of 1800mm²/s at 25 °C and a refractive index of 1.460 at 25 °C.

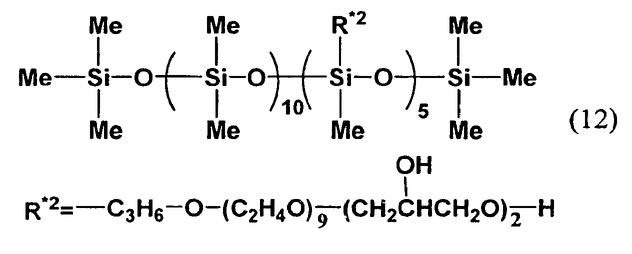

### Example IV

In a reactor, 192 g of organohydrogenpolysiloxane represented by the aforesaid average compositional formula (8), 73 g of polyoxyalkylene group-containing compound represented by the aforesaid average compositional formula (11) and 185 g of ethanol were mixed, to which 0.04 g of 3% solution of chloroplatinic acid in ethanol was added. The mixture was then subjected to a reaction under reflux for 8 hours. Subsequently, the reaction solution was vacuum distilled and then filtered, whereby a silicone represented by the formula (13) shown below was obtained. The silicone was light brown and transparent liquid which had a viscosity of 30,000 mm²/s at 25 °C and a refractive index of 1.423 at 25 °C. 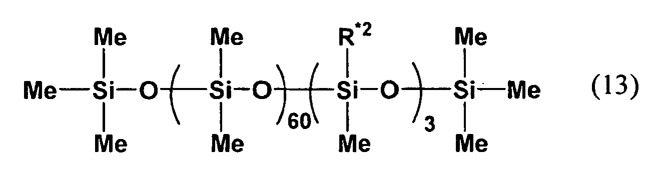 In the formula (13), R^{*2} is as defined above.

### Example V

In a reactor, 65.6 g of organohydrogenpolysiloxane represented by the following formula (14): 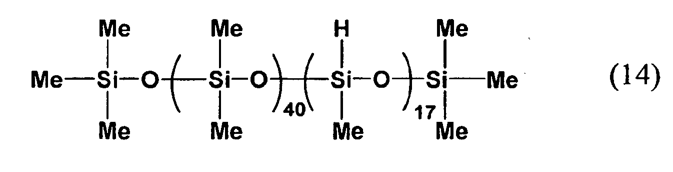 24 g of organopolysiloxane represented by the following formula (15): 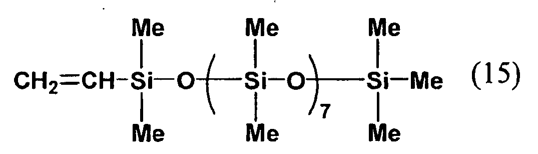 10.8 g of 1-dodecene and 27 g of the polyoxyalkylene group-containing compound of the above formula (11) and 30 g of isopropyl alcohol were mixed, to which 0.03 g of 3% solution of chloroplatinic acid in ethanol was added. The mixture was then subjected to a reaction under reflux for 5 hours. Subsequently, the reaction solution was vacuum distilled, whereby a silicone represented by the formula (16) shown below was obtained. The silicone was pale yellow and transparent liquid which had a viscosity of 2500 mm²/s at 25 °C and a refractive index of 1.433 at 25 °C. 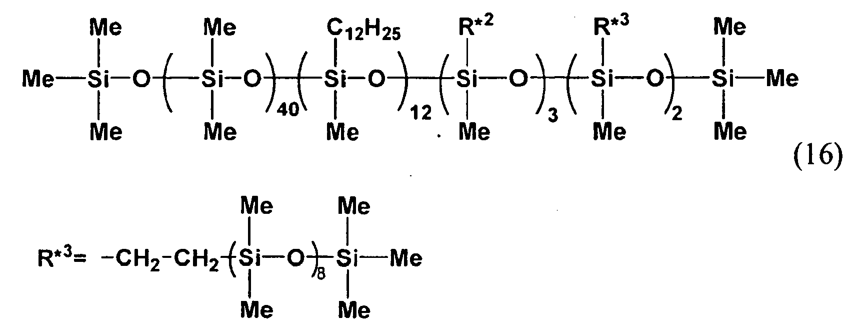 In the formula (16), R^{*2} is as defined above.

### Example VI

In a reactor, 120 g of organohydrogenpolysiloxane represented by the following formula (17): 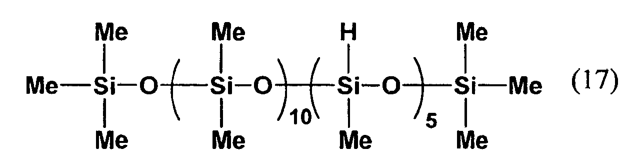 84 g of organopolysiloxane represented by the aforesaid formula (15), 51 g of 1-dodecene, 60 g of polyoxyalkylene group-containing compound of the aforesaid formula (11), and 95 g of isopropyl alcohol were mixed, to which 0.05 g of 3% solution of chloroplatinic acid in ethanol was added. The mixture was then subjected to a reaction under reflux for 5 hours. Subsequently, the reaction solution was vacuum distilled, whereby the silicone represented by the formula (18) shown below was obtained. The silicone was pale yellow and transparent liquid which had a viscosity of 710mm²/s at 25 °C and a refractive index of 1.432. 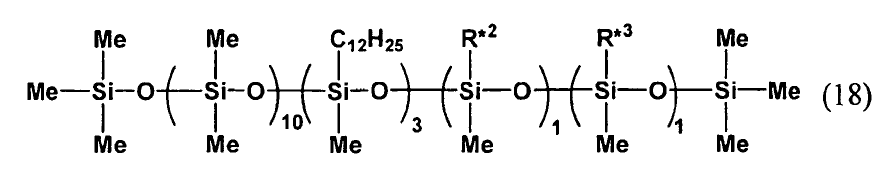 In the formula (18), R^{*2} and R^{*3} are as defined above.

### Example VII

In a reactor, 217.6 g of organohydrogenpolysiloxane represented by the following formula (19): 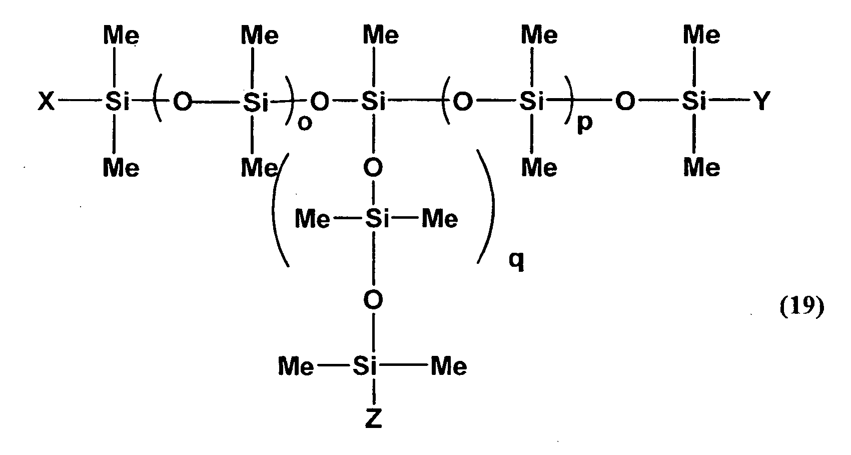 60 g of vinyl group-containing polyoxyalkylene group-containing compound of the aforesaid formula (11), and 110 g of isopropyl alcohol were mixed, to which 0.04 g of 3% solution of chloroplatinic acid in ethanol was added. The mixture was then subjected to a reaction under reflux for 5 hours. Subsequently, the reaction solution was vacuum distilled, whereby a silicone represented by the following formula (20) was obtained. The silicone was pale yellow and transparent liquid which had a viscosity of 710 mm²/s at 25 °C and a refractive index of 1.417 at 25 °C.

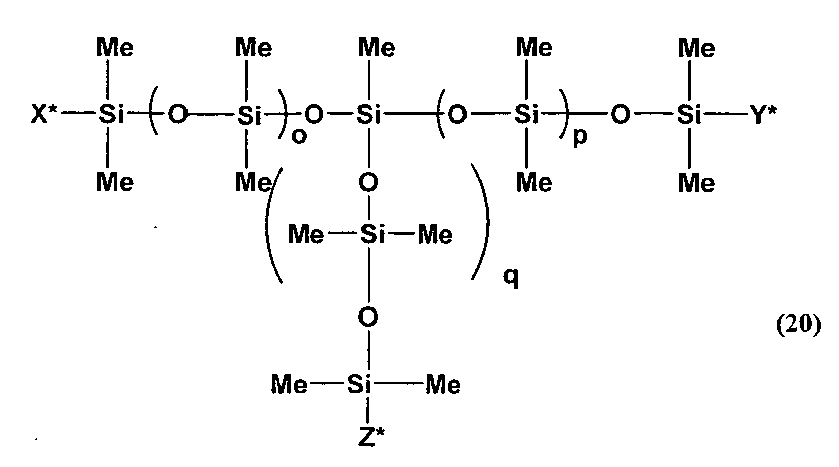

In the formulas (19), a sum of o, p and q is 55 on average, and one of X, Y and Z is a methyl group and the other two are hydrogen atoms. In the formulas (20), a sum of o, p and q is 55 on average, and one of X^{*}, Y^{*} and Z^{*} is a methyl group and the other two are aforesaid R^{*2'}s.

### Comparative Example I

In a reactor, 50 g of organohydrogenpolysiloxane represented by the above formula (5), 97 g of polyglycerin allyl ether and 90 g of isopropyl alcohol were mixed, to which 0.03 g of 3% solution of chloroplatinic acid in ethanol was added. The mixture was then subjected to a reaction under reflux for 8 hours. To the reaction mixture, 23 g of 0.005N aqueous solution of hydrochloric acid was added to hydrolyze unreacted ally ether group and then was neutralized with 0.3 g of 5% sodium bicarbonate solution. Subsequently, the reaction mixture was vacuum distilled and then filtered, whereby a silicone represented by the formula (21) shown below was obtained. The silicone was pale yellow and transparent liquid which had a viscosity of 430,000 mm²/s at 25 °C and a refractive index of 1.469 at 25 °C.

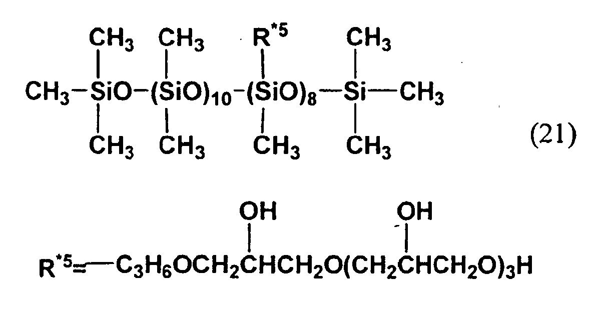

### Comparative Example II

In a reactor, 160 g of organohydrogenpolysiloxane represented by the aforesaid formula (8), 30 g of polyglycerin allyl ether and 133 g of ethyl alcohol were mixed, to which 0.04 g of 3% solution of chloroplatinic acid in ethanol was added. The mixture was then subjected to a reaction under reflux for 8 hours. Subsequently, the reaction mixture was vacuum distilled and then filtered, whereby a silicone represented by the formula (22) shown below was obtained. The silicone was cloudy and viscous white substance which had a viscosity of 130,000 mm²/s at 25 °C and a refractive index of 1.417 at 25 °C. 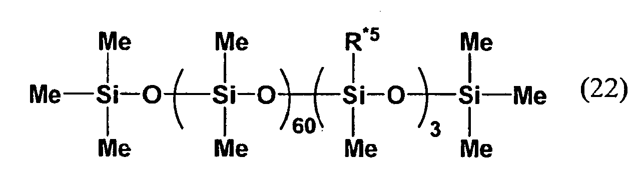 In the formula (22), R^{*5} is as defined above.

### Comparative Example III

In a reactor, 360 g of organohydrogenpolysiloxane represented by the aforesaid formula (10), 700 g of polyoxyalkylene group-containing compound represented by the following formula (23):

**CH₂=CH-CH₂-O-(C₂H₄O)₉-H** (23)

and 320g of isopropyl alcohol were mixed, to which 0.2 g of 3% solution of chloroplatinic acid in ethanol was added. The mixture was then subjected to a reaction under reflux for 8 hours. Subsequently, the reaction mixture was vacuum distilled , whereby a silicone represented by the following formula (24) was obtained. The silicone was pale yellow and transparent liquid which had a viscosity of 510 mm²/s at 25 °C and a refractive index of 1.454 at 25 °C.

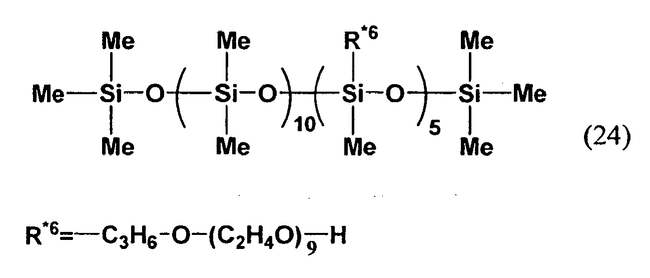

### Comparative Example IV

In a reactor, 130 g of organohydrogenpolysiloxane represented by the aforesaid formula (8), 40 g of polyoxyalkylene group-containing compound represented by the aforesaid formula (23), and 43g of isopropyl alcohol were mixed, to which 0.03 g of 3% solution of chloroplatinic acid in ethanol was added. The mixture was then subjected to a reaction under reflux for 8 hours. Subsequently, the reaction mixture was vacuum distilled and then filtered, whereby a silicone represented by the formula (25) shown below was obtained. The silicone was colorless transparent liquid which had a viscosity of 820 mm²/s at 25 °C and a refractive index of 1.419 at 25 °C. 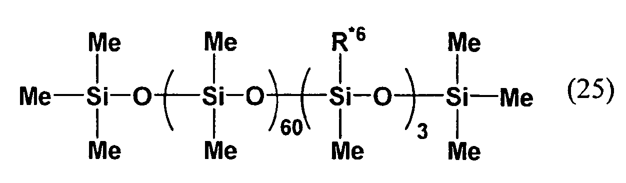 In the formula (25), R^{*6} is as defined above.

### Comparative Example V

In a reactor, 165 g of organohydrogenpolysiloxane represented by the aforesaid formula (5), 230 g of polyoxyalkylene group-containing compound represented by the aforesaid formula (23), 66g of glycerin monoally ether and 280 g of isopropyl alcohol were mixed, to which 0.14 g of 3% solution of chloroplatinic acid in ethanol was added. The mixture was then subjected to a reaction under reflux for 16 hours. Subsequently, the reaction mixture was vacuum distilled , whereby a silicone represented by the formula (26) shown below was obtained. It was yellow and cloudy liquid which had a viscosity of 790 mm²/s at 25 °C and a refractive index of 1.448 at 25 °C. 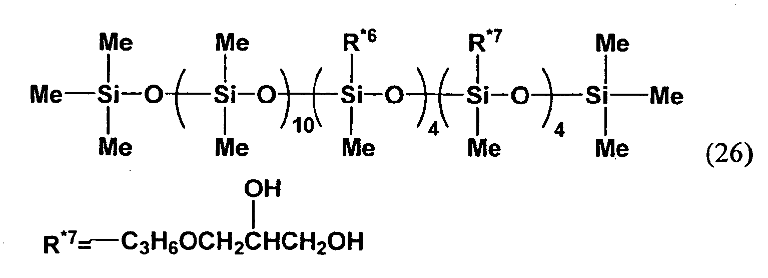 In the formula (26), R^{*5} is as defined above.

### Comparative Example VI

In a reactor, 985 g of organohydrogenpolysiloxane represented by the following formula (27): 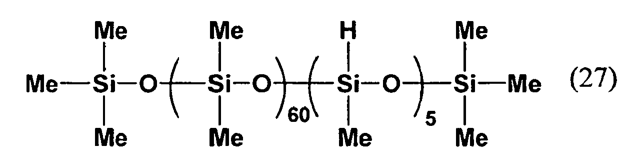 1400 g of polyoxyalkylene group-containing compound represented by the aforesaid formula (23), 300g of glycerin monoally ether and 1400 g of ethyl alcohol were mixed, to which 0.5 g of 3% solution of chloroplatinic acid in ethanol was added. The mixture was then subjected to a reaction under reflux for 12 hours. Subsequently, the reaction mixture was vacuum distilled, whereby a silicone represented by the following formula (28) was obtained. It was light brown and transparent liquid which had a viscosity of 3200 mm²/s at 25 °C and a refractive index of 1.422. 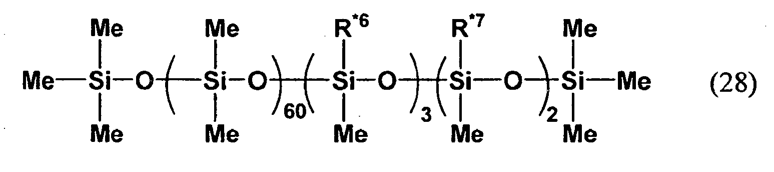 In the formula (28), R^{*6} and R^{*7} are as defined above.

### Evaluation of solubility in water

Aqueous solutions of the silicones prepared in Examples I, III, Comparative Examples I, III and V at a concentration of 1 % were prepared. Appearance of the solutions after left standing at 25 °C or 60 °C for 24 hours was visually observed and rated according to the following rating scale:
A: Transparent;
B: From slightly cloudy to distinctly cloudy;
C: Separated in phases.

Results are as shown in Table 1 wherein "Ex." stands for "Example" and "Comp.Ex." for "Comparative Example."

An aliquot of each aqueous solution was applied to the skin and rated in terms of sensory feels according to the following rating scales. Results are as shown in Table 2.

**Non-tackiness during application and after applied**

| | |
|---|---|
| Not tacky and smooth | A |
| Not tacky | B |
| Uncomfortably tacky | C |

**Affinity for the skin during application and after applied**

| | |
|---|---|
| Very affinitive to give moisturized feel | A |
| Affinitive | B |
| A little affinitive | C |
| Not affinitive | D |

**Non-twitchiness during application and after application**

| | |
|---|---|
| Not twitchy with comfortable smoothness | A |
| Not twitchy with a little smoothness | B |
| Not twitchy with no smoothness | C |
| Twitchy | D |

**Table 1**

| | Ex.I | Ex.III | Comp.Ex.I | Comp.Ex.III | Comp.Ex.V |
|---|---|---|---|---|---|
| 25°C | A | A | B | A | A |
| 60°C | A | A | B | C | B |

**Table 2**

| Evaluation items | Ex.1 | Ex.III | Comp.Ex. | Comp.Ex. III | Comp.Ex_{.} V |
|---|---|---|---|---|---|
| Non-tackiness during application | A | B | B | B | B |
| Affinity for the skin during application | A | B | B | D | C |
| Non-twitchiness during application | A | A | A | C | C |
| Non-tackiness after applied | A | B | B | B | B |
| Affinity for the skin after applied | A | B | B | C | C |
| Non-twitchiness after applied | A | A | A | D | C |
| Overall rating | A | A | B | C | C |

As is evident from the above tables, the silicones of Examples I and III were superior to those of Comparative Examples I, III and V in solubility in water, and thermal stability thereof. Moreover, the silicones of Examples I and III were excellent in sensory feels during and after application to the skin.

### Example 1: W/O Emulsion, evaluation of stability of

### emulsion

W/O emulsions were prepared according to the formulation shown in Table 3 and were evaluated in terms of stability with temperature and time.

**Table 3**

| Components | Ex.1 | Comp.Ex.1 | Comp.Ex.2 |
|---|---|---|---|
| Dimethylpolysiloxane (6 mm²/sec) | 18 | 18 | 18 |
| 1,3-butyleneglycol | 7 | 7 | 7 |
| Silicone of Example II | 2 | | |
| Silicone of Comparative Example IV | | 2 | |
| Silicone of Comparative Example VI | | | 2 |
| NaCl | 0.5 | 0.5 | 0.5 |
| Purified water | 72.5 | 72.5 | 72.5 |

### <Evaluation>

An aliquot of each emulsion was applied to the skin and rated in terms of non-tackiness, affinity, and non-twitchiness during and after application according to the aforesaid rating scales.

**Table 4**

| Evaluation items | Ex.1 | Comp.Ex.1 | Comp.Ex.2 |
|---|---|---|---|
| Non-tackiness during application | A | B | B |
| Affinity for the skin during application | A | D | D |
| Non-twitchiness during application | A | C | C |
| Non-tackiness after applied | A | B | B |
| Affinity for the skin after applied | A | C | C |
| Non-twitchiness after applied | A | D | C |
| Overall rating | A | C | C |

Change in viscosity of each emulsion was determined by measuring viscosity before and after keeping the emulsion at 35 °C for 1, 3 or 5 days. Ratios of the viscosities measured to an initial viscosity were calculated.

**Table 5**

| | Day(s) | | |
|---|---|---|---|
| | 1 | 3 | 5 |
| Example 1 | 0.81 | 0.77 | 0.76 |
| Comparative Example 1 | 0.68 | 0.63 | 0.61 |
| Comparative Example 2 | 0.73 | 0.62 | 0.59 |

As is found from Table 5, the emulsion of Example 1 was stable for several days, whereas the emulsion of Comparative Examples 1 and 2, both comprising the silicone of which side chain lacks a polyether moiety, showed drastic decrease in viscosity in 1 day.

### Examples 2 and 3: Sunscreen agent, evaluation of powder dispersibility

According to the formulation shown in the following Table 6, sunscreen agents were prepared and evaluated. In Table 6, "Ex." stands for example and "Comp.Ex." for comparative example.

**Table 6**

| Components | Ex.2 | Ex.3 | Comp. Ex.3 |
|---|---|---|---|
| 1. Dimethylpolysiloxane (6 mm²/sec) | 5.0 | 6.0 | 6.0 |
| 2. Crosslinked polyether-modified silicone ¹⁾ | 5.0 | 5.0 | 5.0 |
| 3. Glyceryl trioctanoate | 3.0 | 3.0 | 3.0 |
| 4. Polyether-modified silicone ²⁾ | 1.0 | 1.0 | 1.0 |
| 5. Octyl methoxycinnamate | 6.0 | 6.0 | 6.0 |
| 6. Sodium chloride | 0.5 | 0.5 | 0.5 |
| 7. 1,3-butyleneglycol | 2.0 | 2.0 | 2.0 |
| 8. Purified water | Balance | Balance | Balance |
| 9. Perfume | q.s. | q.s. | q.s. |
| 10. Dispersion of zinc oxide(a) | 50 | - | - |
| 11. Dispersion of zinc oxide(b) | - | 50 | - |
| 12. Dispersion of zinc oxide(c) | - | - | 50 |
| Evaluation results | | | |
| 1. Stability of dispersion | A | A | C |
| 2. Sensory feels:Smoothness | A | A | B |
| Spreadability | A | B | B |
| Translucency of applied sunscreen agent | A | A | C |
| Non-tackiness | A | A | C |
| Sunscreening effect | A | A | B |

| | | | |
|---|---|---|---|
| 1)KSG-210 from Shin-Etsu Chemical Co., Ltd 2)KF-6019 from Shin-Etsu Chemical Co., Ltd | | | |

### Preparation of dispersion of zinc oxide

In 40 g of decamethylcyclopentasiloxane, 10 g of each of the following silicone compounds was dissolved.
(a)Silicone compound prepared in Example II
(b)Silicone compound prepared in Example IV
(c)Silicone compound prepared in Comparative Example II

In the solution thus obtained, 50 g of zinc oxide, MZ505S, ex Tayca Co., was dispersed with a beads mill.

### Preparation procedures of the sunscreen agent

A: Components 1 to 5 were mixed.
B: Components 6 to 8 were mixed.
C: To the homogeneous mixture prepared in the step A, the homogeneous mixture prepared in the step B was added and emulsified.
D: To the emulsion, Components 9 to 12 were added, whereby sunscreen agent was obtained.

The sunscreen agents thus prepared were evaluated according to the following methods.

### Stability of powder dispersion

The sunscreen agents were left standing at room temperature for 1 month. Then, they were visually observed for agglomeration of powder and rated on the scale shown below.

**Rating scale**

| | |
|---|---|
| No agglomerated powder is observed. | A |
| Possibly agglomerated powder is observed. | B |
| Some agglomerated powder is observed. | C |
| Agglomerated powder is clearly observed. | D |

### Sensory evaluation

The sunscreen agents were rated by 50 women panelists in terms of smooth feel, spreadability, translucency of applied sunscreen agent, non-tackiness, and sunscreening effect on a scale of 1 to 5 points as shown below, and then the points were averaged.

| | |
|---|---|
| 5 points | Excellent |
| 4 points | Good |
| 3 points | Average |
| 2 points | Slightly bad |
| 1 point | Bad |

| Averaged points | Overall rating |
|---|---|
| 4.5 or higher | A |
| 3.5 or higher to lower than 4.5 | B |
| 2.5 or higher to lower than 3.5 | C |
| 1.5 or higher to lower than 2.5 | D |

As shown in Table 6, the sunscreen agents of Examples 2 and 3 showed no agglomeration of powder and were rated high in sensory tests. In contrast, the sunscreen agent of Comparative Example 3, which contained zinc oxide powder treated with a silicone of which side chain lacks a polyether moiety, clearly showed agglomerated powder which caused inferior translucency of the applied sunscreen agent and sensory feels.

The followings are examples of various cosmetics. Stability of the cosmetics with time was tested by putting an aliquot of cosmetic in a closed container, standing the container in a thermostatic chamber at 25 °C for 1 month, and then observing appearance of the cosmetic.

### Example 4: Cream

| Components | % |
|---|---|
| 1. Ethyl alcohol | 17.0 |
| 2. Propylene glycol | 3.0 |
| 3. Silicone (Example I) | 0.5 |
| 4. Glyceryl trioctanoate | 2.0 |
| 5. Sericite treated with silicone¹⁾ | 0.2 |
| 6. Composite of hybrid silicone²⁾ | 0.5 |
| 7. Carboxyvinyl polymer (1% aqueous solution ) | 20.0 |
| 8. Xanthan gum (2% aqueous solution) | 6.0 |
| 9. Triethanolamine | 0.2 |
| 10. Antiseptics | q.s. |
| 11. Perfume | q.s. |
| 12. Purified water | q.s. |

| | |
|---|---|
| 1) Sericite was heat-treated with 2% KF-9909 from Shin-Etsu Chemical Co., Ltd. 2)KSP-100 from Shin-Etsu Chemical Co., Ltd. | |

### Preparation procedures

A: Components 1 to 6 were mixed.
B: Components 7 to 12 were mixed and dissolved.
C: To the solution prepared in the step B, the mixture prepared in step A was added and mixed.

The cream thus obtained had fine texture, good spreadability, non-tackiness, non-greasiness and gave moisturized and refreshing feel to the skin. The applied cream stayed long. It was stabile with time and temperature.

### Example 5:O/W type cream

| Components | % |
|---|---|
| 1. Crosslinked dimethylpolysiloxane¹⁾ | 10.0 |
| 2. Glyceryl trioctanoate | 5.0 |
| 3. Dipropylene glycol | 7.0 |
| 4. Glycerin | 5.0 |
| 5. Methylcellulose(2% aqueous solution)²⁾ | 7.0 |
| 6. Polyacrylamide emulsifier³⁾ | 2.0 |
| 7. Mica/titanium dioxide treated with silicone⁴⁾ | 1.0 |
| 8. Antiseptics | q.s. |
| 9. Perfume | q.s. |
| 10. Purified water | q.s. |

| | |
|---|---|
| 1) KSG-16from Shin-Etsu Chemical Co., Ltd. 2) Metholose SM-4000from Shin-Etsu Chemical Co., Ltd. 3) Sepigel from Seppic.Co. 4) Mica/titanium dioxide was heat-treated with 2% solution of the silicone prepared in Example II. | |

### Preparation procedures

A: Components 3 to 10 were mixed.
B: Components 1 and 2 were mixed. The mixture thus obtained was added to the mixture prepared in the step A and emulsified by stirring.

The O/W type cream thus obtained had fine texture, good spreadability, non-tackiness, and non-greasiness. It gave moisturized and refreshing feel to the skin and the applied cream stayed long. It was stabile with time and temperature, too.

### Example 6:O/W type cream

| Components | % |
|---|---|
| 1. Crosslinked alkyl-modified silicone¹⁾ | 8.0 |
| 2. Crosslinked methylphenylpolysiloxane²⁾ | 2.0 |
| 3. Silicone (Example I) | 0.5 |
| 4. Isododecyl isononanate | 5.0 |
| 5. Dipropylene glycol | 7.0 |
| 6. Glycerin | 5.0 |
| 7. Methylcellulose(2% aqueous solution³⁾ | 7.0 |
| 8. Polyacrylamide emulsifier⁴⁾ | 1.5 |
| 9. Guanine | 1.0 |
| 10. Antiseptics | q.s. |
| 11. Perfume | q.s. |
| 12. Purified water | 63.0 |

| | |
|---|---|
| 1) KSG-43 from Shin-Etsu Chemical Co., Ltd. 2) KSG-18A from Shin-Etsu Chemical Co., Ltd. 3) Metholose SM-4000 from Shin-Etsu Chemical Co., Ltd. 4) Sepigel from Seppic.Co. | |

### Preparation procedures

A: Components 3,5 to 12 were mixed.
B: Components 1, 2 and 4 were mixed.
C: To the mixture prepared in the step B, the mixture prepared in the step A was added and emulsified by stirring.

The O/W type cream thus obtained had fine texture, good spreadability, non-tackiness, and non-greasiness. It gave moisturized and refreshing feel to the skin and the applied cream stayed long. It was stabile with time and temperature, too.

### Example 7:O/W type cream

| Components | % |
|---|---|
| 1. Crosslinked alkyl-modified silicones¹⁾ | 2.0 |
| 2. Crosslinked dimethylpolysiloxane²⁾ | 15.0 |
| 3. Decamethylcyclopentasiloxane | 10.0 |
| 4. Dimethylpolysiloxane (6 mm²/sec) | 18.0 |
| 5. Polyether-modified silicone³⁾ | 0.4 |
| 6. Silicone (Example III) | 0.3 |
| 7. Propylene glycol | 3.0 |
| 8. Polyacrylamide emulsifier⁴⁾ | 0.8 |
| 9. Xanthan gum (2% aqueous solution) | 8.0 |
| 10. Antiseptics | q.s. |
| 11. Perfume | q.s. |
| 12. Purified water | 42.5 |

| | |
|---|---|
| 1) KSG-42 from Shin-Etsu Chemical Co., Ltd. 2) KSG-16 from Shin-Etsu Chemical Co., Ltd. 3) KF-6011 from Shin-Etsu Chemical Co., Ltd. 4) Sepigel from Seppic.Co. | |

### Preparation procedures

A: Components 1 to 4 were mixed.
B: Components 5 to 12 were mixed.
C: To the mixture prepared in the step B, the mixture prepared in the step A was added and emulsified by stirring.

The O/W type cream thus obtained had fine texture, good spreadability, non-tackiness, and non-greasiness. It gave moisturized and refreshing feel to the skin and the applied cream stayed long. It was stabile with time and temperature.

### Example 8:O/W type UV cut-off cream

| Components | % |
|---|---|
| 1. Crosslinked methylphenylpolysiloxane¹⁾ | 5.0 |
| 2. Cetyl isooctanoate | 5.0 |
| 3. Crosslinked alkyl-modified silicone²⁾ | 1.0 |
| 4. Titanium oxide dispersed in | |
| decamethylcyclopentasiloxane³⁾ | 15.0 |
| 5. Polyether-modified silicone ⁴⁾ | 1.0 |
| 6. Silicone (Example III) | 1.0 |
| 7. Polyacrylamide emulsifier⁵⁾ | 2.0 |
| 8. Propylene glycol | 5.0 |
| 9. Methylcellulose(2% aqueous solution)⁶⁾ | 5.0 |
| 10. Antiseptics | q.s. |
| 11. Perfume | q.s. |
| 12. Purified water | 60.0 |

| | |
|---|---|
| 1) KSG-18A from Shin-Etsu Chemical Co., Ltd. 2) KSG-43 from Shin-Etsu Chemical Co., Ltd. 3) SPD-T5 from Shin-Etsu Chemical Co., Ltd. 4) KF-6011 from Shin-Etsu Chemical Co., Ltd. 5) Sepigel from Seppic.Co. 6) MetholoseSM-4000 from Shin-Etsu Chemical Co., Ltd. | |

### Preparation procedures

A: Components 5 to 8, 10 and 12 were mixed.
B: Components 1 to 3 were mixed and added to the mixture prepared in the step A and emulsified.
C: To the emulsion prepared in the step B, Component 4, and then Components 9 and 11 were added.

The UV protective cream thus obtained spread smoothly on the skin to give non-tackiness, and non-greasiness feel. The applied cream gave translucent finish and stayed long. It was stabile with time and temperature.

### Example 9: Facial wash

| Components | % |
|---|---|
| 1. Polyoxyethylene(6) lauryl ether¹⁾ | 5.0 |
| 2. Silicone (Example I) | 10.0 |
| 3. Ethyl alcohol | 10.0 |
| 4. Lauryl dimethylamine oxide²⁾ | 2.0 |
| 5. Propylene glycol | 3.0 |
| 6. Sodium citrate | 0.5 |
| 7. Antiseptics | q.s. |
| 8. Perfume | q.s. |
| 9. Purified water | 69.5 |

| | |
|---|---|
| 1)Pegnol L-6 from Toho Chemical Industry Co.,Ltd. 2)Unisafe A-LM from NOF Corp. | |

### Preparation procedures

A: Components 1 to 7 and 9 were mixed and dissolved.
B: To the homogeneous solution prepared in the step A, Component 8 was added.

The facial wash thus obtained was miscible with a cosmetic and sebum to clean them well. It spread smoothly on the skin and gave moisturized feel after use.

### Example 10: Makeup remover

| Components | % |
|---|---|
| 1. Polyoxyethylene(10) Sorbitan monooreate | 10.0 |
| 2. Silicone (Example I) | 20.0 |
| 3. Sorbitol | 10.0 |
| 4. Carrageenan | 0.5 |
| 5. Glycerin | 5.0 |
| 6. Sodium citrate | 0.5 |
| 7. Antiseptics | q.s. |
| 8. Perfume | q.s. |
| 9. Purified water | 54.0 |

### Preparation procedures

A: Components 1 to 7 were dissolved in Component 9.
B: To the homogeneous solution prepared in the step A, Component 8 was added.

The makeup remover was used to remove foundation and found that it was miscible with foundation to remove it very well. It spread smoothly on the skin and gave moisturized feel after use.

### Example 11: Suntan cream

| Components | % |
|---|---|
| 1. Decamethylcyclopentasiloxane | 15.0 |
| 2. Dimethylpolysiloxane (6 mm²/sec) | 5.0 |
| 3. Stearyl-modified acryl silicone¹⁾ | 0.5 |
| 4. Silicone (Example IV) | 5.0 |
| 5. Palmitic acid | 0.2 |
| 6. Dimethyloctyl p-aminobenzoate | 0.5 |
| 7. 4-t-butyl-4'-methoxybenzoylmethane | 0.5 |
| 8. Kaolin | 0.5 |
| 9. Iron oxide red | 0.2 |
| 10. Iron oxide yellow | 0.3 |
| 11. Iron oxide black | 0.1 |
| 12. Mica coated with titanium oxide | 1.0 |
| 13. Sodium L-glutamate | 3.0 |
| 14. 1,3-butylene glycol | 5.0 |
| 15. Dioctadecyldimethylammonium chloride | 0.1 |
| 16. Antioxidant | q.s. |
| 17. Antiseptics | q.s. |
| 18. Perfume | q.s. |
| 19. Purified water | Balance |

| | |
|---|---|
| 1) KP-561P from Shin-Etsu Chemical Co., Ltd. | |

### Preparation procedures

A: Components 1 to 7, 16 and 17 were heat-mixed.
B: Component 15 and a part of Component 19 were stirred while heated, to which Components 8 to 12 were added and dispersed.
C: Components 13, 14 and the rest of Component 19 were mixed. Solution thus obtained was mixed with the dispersion prepared in the step B.

The suntan cream thus obtained had fine texture. It spread smoothly to give non-greasy, moisturized, and refreshing feels to the skin. It was confirmed to be stable with time.

### Example 12: Sunscreen cream

| Components | % |
|---|---|
| 1. Decamethylcyclopentasiloxane | 17.5 |
| 2. Acrylic silicone | |
| resin/decamethylcyclopentasiloxane¹⁾ | 12.0 |
| 3. Glyceryl triisooctanoate | 5.0 |
| 4. Octyl paramethoxycinnamate | 6.0 |
| 5. Crosslinked polyether-modified silicone²⁾ | 5.0 |
| 6. Silicone (Example V) | 1.0 |
| 7. Lipophilized zinc oxide³⁾ | 20.0 |
| 8. Sodium chloride | 0.5 |
| 9. 1,3-butylene glycol | 2.0 |
| 10. Antiseptics | q.s. |
| 11. Perfume | q.s. |
| 12. Purified water | 31.0 |

| | |
|---|---|
| 1) KP-545 from Shin-Etsu Chemical Co., Ltd. 2) KSG-240 from Shin-Etsu Chemical Co., Ltd. 3) Zinc oxide was heat-treated with AES-8303 from Shin-Etsu Chemical Co., Ltd. in an amount of 5 5% of a weight of zinc oxide, | |

### Preparation procedures

A: A part of Component 1 and Component 2 were mixed, to which Component 7 was added and dispersed with a bead mill.
B: The rest of Component 1 and Components 3 to 6 were mixed.
C: Components 8 to 10 and 12 were mixed.
D: To the homogeneous mixture prepared in the step B, the homogeneous mixture prepared in the step C was added and emulsified. To the emulsion thus obtained, the dispersion prepared in the step A and Component 11 were added.

The sunscreen cream thus obtained was non-tacky and spread smoothly to give gloss finish. It lasted long with good affinity for the skin, and was stable with temperature and time.

### Example 13: Sunscreen milky lotion

| Components | % |
|---|---|
| 1. Decamethylcyclopentasiloxane | 3.5 |
| 2. Dimethylpolysiloxane (6 mm²/sec) | 5.0 |
| 3. Glyceryl triisooctanoate | 5.0 |
| 4. Silicone (Example II) | 1.0 |
| 5. Crosslinked polyether-modified silicone¹⁾ | 3.0 |
| 6. Titanium oxide dispersed in | |
| decamethylcyclopentasiloxane²⁾ | 25.5 |
| 7. Zinc oxide dispersed in | |
| decamethylcyclopentasiloxane³⁾ | 35.5 |
| 8. Dipropylene glycol | 3.0 |
| 9. Sodium citrate | 0.5 |
| 10. Antiseptics | q.s. |
| 11. Perfume | q.s. |
| 12. Purified water | 19.5 |

| | |
|---|---|
| 1) KSG-210 from Shin-Etsu Chemical Co., Ltd. 2) SPD-T5 from Shin-Etsu Chemical Co., Ltd. 3) SPD-Z5 from Shin-Etsu Chemical Co., Ltd. | |

### Preparation procedures

A: Components 1 to 5 were mixed.
B: Components 8 to 10 were dissolved in Component 12.
C: To the homogeneous mixture prepared in the step A, the solution prepared in the step B was added and emulsified, to which Components 6, 7 and 11 were added.

The sunscreen milky lotion thus obtained was non-tacky and spread smoothly to give gloss finish. It lasted long with good affinity for the skin, and was stable with temperature and time.

### Example 14: Sunscreen milky lotion

| Components | % |
|---|---|
| 1. Decamethylcyclopentasiloxane | 3.0 |
| 2. Dimethylpolysiloxane (6 mm²/sec) | 3.0 |
| 3. Glyceryl trioctanoate | 2.0 |
| 4. Crosslinked glycrin-modified silicone¹⁾ | 3.0 |
| 5. Crosslinked dimethylpolysiloxane²⁾ | 2.0 |
| 6. Silicone (Example II) | 1.0 |
| 7. Zinc oxide dispersed in | |
| decamethylcyclopentasiloxane³⁾ | 35.5 |
| 8. Titanium oxide dispersed in | |
| decamethylcyclopentasiloxane⁴⁾ | 25.5 |
| 9. Sodium citrate | 0.2 |
| 10. Dipropylene glycol | 3.0 |
| 11. Antiseptics | q.s. |
| 12. Perfume | q.s. |
| 13. Purified water | Balance |

| | |
|---|---|
| 1) KSG-710 from Shin-Etsu Chemical Co., Ltd. 2) KSG-15 from Shin-Etsu Chemical Co., Ltd. 3) SPD-Z6 from Shin-Etsu Chemical Co., Ltd. 4) SPD-T5 from Shin-Etsu Chemical Co., Ltd. | |

### Preparation procedures

A: Components 1 to 6 were mixed.
B: Components 9 to 11 were dissolved in Component 13.
C: To the homogeneous mixture prepared in the step A, the solution prepared in the step B was added and emulsified, to which Components 7, 8 and 12 were added.

The sunscreen milky lotion thus obtained was non-tacky and spread smoothly to give gloss finish. It lasted long with good affinity for the skin, and was stable with temperature and time.

### Example 15:W/O type hand cream

| Components | % |
|---|---|
| 1. Decamethylcyclopentasiloxane | 30.0 |
| 2. Liquid paraffin | 10.0 |
| 3. Amino-modified silicone gum¹⁾ | 15.0 |
| 4. Silicone (Example IV) | 4.0 |
| 5. Distearyldimethylammonium chloride | 0.8 |
| 6. Vitamin E acetate | 0.1 |
| 7. Polyethylene glycol 4000 | 1.0 |
| 8. Glycerin | 10.0 |
| 9. Smectone | 1.2 |
| 10. Antiseptics | q.s. |
| 11. Perfume | q.s. |
| 12. Purified water | Balance |

| | |
|---|---|
| 1) Amine equivalent 70,000g/mol | |

### Preparation procedures

A: Components 1 and 3 were heat-mixed, to which Components 2, 4, 5, 6 and 10 were added while heating the mixture.
B: Components 7 to 9 and Component 12 were heat-mixed.
C: To the mixture prepared in the step A, the mixture prepared in the step B was gradually added and emulsified. The emulsion thus obtained was cooled to which Component 11 was added.

The hand cream thus obtained was non-tacky and spread smoothly to give moisturized feel. It effectively protected the skin, and was stable with time.

### Example 16:O/W type hand cream

| Components | % |
|---|---|
| 1. Acrylic silicone resin/decamethylcyclopentasiloxane¹⁾ | 10.0 |
| 2. Stearyl-modified acrylic silicone resin²⁾ | 8.0 |
| 3. Cetanol | 1.0 |
| 4. Glyceryl triisostearate | 5.0 |
| 5. Stearic acid | 3.0 |
| 6. Glyceryl monostearate | 1.5 |
| 7. Silicone (Example I) | 0.7 |
| 8. Sorbitan sesquioleate | 0.5 |
| 9. Polyoxyethylenesorbitan monooleate | 1.0 |
| 10. Sodium hydroxide (1% aqueous solution) | 10.0 |
| 11. 1,3-butylene glycol | 5.0 |
| 12. Antiseptics | q.s. |
| 13. Perfume | q.s. |
| 14. Purified water | 54.3 |

| | |
|---|---|
| 1) KP-545 from Shin-Etsu Chemical Co., Ltd. 2) KP-561P from Shin-Etsu Chemical Co., Ltd. | |

### Preparation procedures

A: Components 1 to 9 were heat-mixed.
B: Components 10 to 12 and 14 were heat-mixed.
C: To the mixture prepared in the step A, the mixture prepared in the step B was added and emulsified. The emulsion thus obtained was cooled to which Component 13 was added.

The O/W hand cream thus obtained was non-tacky and spread smoothly to give gloss finish. It lasted long with good affinity for the skin, and was stable with temperature and time.

### Example 17:Moisturizing cream

| Components | % |
|---|---|
| 1. Decamethylcyclopentasiloxane | 10.0 |
| 2. Methylphenylpolysiloxane | 3.0 |
| 3. Liquid paraffin | 5.0 |
| 4. Stearoxy-modified silicone¹⁾ | 8.0 |
| 5. Silicone (Example II) | 2.0 |
| 6. Organopolysiloxane elastomer spherical powder²⁾ | 2.5 |
| 7. Hydrophobic silica³⁾ | 2.0 |
| 8. Zinc stearate | 2.0 |
| 9. Vitamin E acetate | 3.0 |
| 10. Polyethylene glycol 400 | 1.0 |
| 11. Sodium lactate | 1.0 |
| 12. 1,3-butylene glycol | 5.0 |
| 13. Antiseptics | q.s. |
| 14. Perfume | q.s. |
| 15. Purified water | 55.5 |

| | |
|---|---|
| 1) KP-7002 from Shin-Etsu Chemical Co., Ltd. 2) KMP-590 from Shin-Etsu Chemical Co., Ltd. 3) Aerosil 972 from Nippon Aerosil Co.,Ltd. | |

### Preparation procedures

A: Components 1 to 5, 8 and 9 were mixed, to which Components 6 and 7 were added and dispersed.
B: Components 10 to 13 were dissolved in Component 15.
C: To the homogeneous dispersion prepared in the step A, the mixture prepared in the step B was slowly added and emulsified. The emulsion thus obtained was cooled to which Component 14 was added.

The moisturizing cream thus obtained was non-tacky and spread smoothly to give moisturized and refreshing feel to the skin. It was confirmed to be stable with temperature and time

### Example 18: Milky lotion

.

| Components | % |
|---|---|
| 1. Decamethylcyclopentasiloxane | 15.0 |
| 2. Methylphenylpolysiloxane | 5.0 |
| 3. Squalane | 5.0 |
| 4. Pentaerythritol tetra-2-ethyl hexanoate | 5.0 |
| 5. Silicone (Example II) | 3.0 |
| 6. Organopolysiloxane elastomer spherical powder¹⁾ | 2.0 |
| 7. Hydrophobic silica²⁾ | 0.5 |
| 8. Magnesium ascorbate phosphate | 1.0 |
| 9. Sodium chloride | 1.0 |
| 10. Polyethylene glycol 11000 | 1.0 |
| 11. Propylene glycol | 8.0 |
| 12. Antiseptics | q.s. |
| 13. Perfume | q.s. |
| 14. Purified water | 53.5 |

| | |
|---|---|
| 1) KMP-590 from Shin-Etsu Chemical Co., Ltd. 2) Aerosil 972 from Nippon Aerosil Co.,Ltd. | |

### Preparation procedures

A: Components 1 to 5 were mixed, to which Components 6 and 7 were added and dispersed.
B: To Component 14, Components 8 to 10 were added and dissolved, to which homogeneous mixture of Components 11 and 12 were added.
C: To the dispersion prepared in the step A, the mixture prepared in the step B was slowly added and emulsified. The emulsion thus obtained was cooled, to which Component 13 was added.

The milky lotion thus obtained was non-tacky and spread smoothly to give moisturized feel to the skin. It was confirmed to be stable with temperature and time.

### Example 19: Beautifying lotion

| Components | % |
|---|---|
| 1. Decamethylcyclopentasiloxane | 12.0 |
| 2. Glyceryl triisooctanoate | 10.0 |
| 3. Silicone (Example IV) | 2.0 |
| 4. Silicone (Example VI) | 0.2 |
| 5. Glycerin | 10.0 |
| 6. Magnesium ascorbate phosphate | 3.0 |
| 7. Sodium chloride | 2.0 |
| 8. Antiseptics | q.s. |
| 9. Perfume | q.s. |
| 10. Purified water | 60.8 |

### Preparation procedures

A: Components 1 to 4 were mixed.
B: Components 5 to 8 were dissolved in Component 10.
C: To the mixture prepared in the step A, the solution prepared in the step B was slowly added and emulsified while stirring, to which Component 9 was added.

The beautifying lotion thus obtained had fine texture and was non-tacky. It spread smoothly to give moisturized feel to the skin. It was confirmed to be stable with temperature and time.

### Example 20:O/W/O type milky lotion

| Components | % |
|---|---|
| 1. Crosslinked polyether-modified silicone¹⁾ | 3.0 |
| 2. Silicone (Example II) | 1.0 |
| 3. Glyceryl triisooctanoate | 14.0 |
| 4. Crosslinked alkyl-modified silicone compound²⁾ | 5.0 |
| 5. Sucrose stearate | 3.0 |
| 6. Glycerin | 5.0 |
| 7. 1,3-butylene glycol | 5.0 |
| 8. Antiseptics | q.s. |
| 9. Purified water | 60.0 |
| 10. Macadamia nut oil | 2.0 |
| 11. Cetyl alcohol | 2.0 |
| 12. Perfume | q.s. |

| | |
|---|---|
| 1)KSG-210 from Shin-Etsu Chemical Co., Ltd. 2)KSG-43 from Shin-Etsu Chemical Co., Ltd. | |

### Preparation procedures

A: Components 1 to 4 were mixed.
B: Components 5 to 9 were heat-mixed.
C: Components 10 to 12 were heat-mixed.
D: To the mixture prepared in the step B, the mixture prepared in the step C was added and emulsified, and then cooled.
E: To the mixture prepared in the step A, the mixture prepared in the step D was added and emulsified.

The milky lotion thus obtained was non-tacky and non-greasy. It spread smoothly to give translucent finish which lasted long. It was confirmed to be stable with temperature and time.

### Example 21:O/W/O type milky lotion

| Components | % |
|---|---|
| 1. Crosslinked alkyl/polyether-modified silicone¹⁾ | 3.0 |
| 2. Silicone (Example V) | 1.0 |
| 3. Glyceryl triisooctanoate | 14.0 |
| 4. Crosslinked alkyl-modified silicone compound²⁾ | 5.0 |
| 5. Sucrose monostearate | 3.0 |
| 6. Glycerin | 5.0 |
| 7. 1,3-butylene glycol | 5.0 |
| 8. Antiseptics | q.s. |
| 9. Purified water | 60.0 |
| 10. Macadamia nut oil | 2.0 |
| 11. Cetyl alcohol | 2.0 |
| 12. Perfume | q.s. |

| | |
|---|---|
| 1) KSG-310 from Shin-Etsu Chemical Co., Ltd. 2) KSG-41 from Shin-Etsu Chemical Co., Ltd. | |

### Preparation procedures

A: Components 1 to 4 were mixed.
B: Components 5 to 9 were heat-mixed.
C: Components 10 to 12 were heat-mixed.
D: The mixture prepared in the step C was added to the mixture prepared in the step B and emulsified while stirring. The emulsion obtained was then cooled.
   E: The emulsion obtained in the step D was added the mixture prepared in the step A and emulsified while stirring.

The milky lotion thus obtained was non-tacky and non-greasy. It spread smoothly to give refreshing feel and translucent finish which lasted long on the skin. It was confirmed to be stable with temperature and time.

### Example 22:O/W/O type liquid foundation

| Components | % |
|---|---|
| 1. Crosslinked polyether-modified silicone¹⁾ | 4.0 |
| 2. Silicone (Example V) | 1.0 |
| 3. Propylene glycol decanoate | 5.0 |
| 4. Isopropyl myristate | 5.0 |
| 5. Pigment | 10.0 |
| 6. Hydrogenated egg yolk-origin phospholipids | 1.0 |
| 7. Glycerin | 2.0 |
| 8. 1,3-butylene glycol | 10.0 |
| 9. Antiseptics | q.s. |
| 10. Purified water | 52.0 |
| 11. Squalane | 5.0 |
| 12. Cetyl alcohol | 5.0 |
| 13. Perfume | q.s. |

| | |
|---|---|
| 1) KSG-210 from Shin-Etsu Chemical Co., Ltd. | |

### Preparation procedures

A: Components 1 to 4 were mixed.
B: Components 5 to 10 were heat-mixed.
C: Components 11 to 13 were heat-mixed.
D: The mixture prepared in the step C was added to the mixture prepared in the step B and emulsified while stirring. The emulsion obtained was then cooled.
   E: To the mixture prepared in the step A, the emulsion obtained in the step D was added and emulsified while stirring.

The liquid foundation thus obtained was non-tacky and non-greasy. It spread smoothly to give refreshing feel and translucent finish which lasted long on the skin. It was confirmed to be stable with temperature and time.

### Example 23:Sunscreen milky lotion

| Components | % |
|---|---|
| 1. Pentaerythritol tetra-2-ethyl hexanoate | 10.0 |
| 2. Cetyl 2-ethyl hexanoate | 5.0 |
| 3. Squalane | 10.0 |
| 4. Silicone (Example II) | 3.0 |
| 5. Octyl paramethoxycinnamate | 2.0 |
| 6. 2,4-dihydroxybenzophenone | 5.0 |
| 7. Composite of hybrid silicone¹⁾ | 1.5 |
| 8. Hydrophobic silica²⁾ | 0.5 |
| 9. Polyethylene glycol 6000 | 1.0 |
| 10. Propylene glycol | 8.0 |
| 11. Antiseptics | q.s. |
| 12. Perfume | q.s. |
| 13. Purified water | Balance |

| | |
|---|---|
| 1) KSP-100 from Shin-Etsu Chemical Co., Ltd. 2) Aerosil 972 from Nippon Aerosil Co.,Ltd. | |

### Preparation procedures

A: In a part of Component 1, Components 5 and 6 were dissolved.
B: The rest of Component 1 and Components 2 to 4 were mixed, to which the solution prepared in the step A, and then Components 7 and 8 were added.
C: Component 9 was dissolved in Component 13, to which homogeneous mixture of Components 10 and 11 was added.
D: The mixture prepared in the step C was slowly added to the mixture prepared in the step B and emulsified, to which Component 12 was added.

The sunscreen lotion thus obtained was non-tacky. It spread smoothly on the skin and was stable with temperature and time.

### Example 24: Liquid foundation

| Components | % |
|---|---|
| 1. Decamethylcyclopentasiloxane | 15.0 |
| 2. Dimethylpolysiloxane (6 mm²/sec) | 5.0 |
| 3. Liquid paraffin | 3.0 |
| 4. Silicone (Example II) | 1.5 |
| 5. Branched polyether-modified | |
| silicone¹⁾ | 1.5 |
| 6. Palmitic acid | 0.5 |
| 7. Hydrophobic silica²⁾ | 5.0 |
| 8. Titanium oxide | 6.0 |
| 9. Iron oxide red | 0.25 |
| 10. Iron oxide yellow | 0.6 |
| 11. Iron oxide black | 0.12 |
| 12. Sericite | 8.03 |
| 13. Dipropylene glycol | 10.0 |
| 14. Magnesium sulfate | 2.0 |
| 15. Antiseptics | q.s. |
| 16. Antioxidant | q.s. |
| 17. Perfume | q.s. |
| 18. Purified water | Balance |

| | |
|---|---|
| 1) KF-6028 from Shin-Etsu Chemical Co., Ltd. 2) Aerosil RY200 from Nippon Aerosil Co., Ltd. | |

### Preparation procedures

A: Components 8 to 12 were mixed.
B: Components 1 to 7 and 16 were mixed at 70 °C, to which the mixture prepared in the step A was added and dispersed.
C: Component 13 to 18 were heated at 70 °C and then added to the dispersion prepared in the step B and emulsified. The emulsion was then cooled to which Component 17 was added.

The liquid foundation thus obtained was non-tacky. It spread smoothly on the skin to give refreshing feel, and applied foundation lasted long on the skin. The emulsified state was stable with time.

### Example 25: Liquid foundation

| Components | % |
|---|---|
| 1. Decamethylcyclopentasiloxane | 16.0 |
| 2. Dimethylpolysiloxane (6 mm²/sec) | 8.0 |
| 3. Octyl paramethoxycinnamate | 3.0 |
| 4. 12-hydroxysteatic acid | 1.0 |
| 5. Silicone (Example II) | 2.0 |
| 6. Fluorine-modified silicone¹⁾ | 5.0 |
| 7. Silicon resin spherical powder²⁾ | 3.0 |
| 8. Titanium oxide fine particles treated with a fluorine compound³⁾ | 8.0 |
| 9. Mica titanium treated with a fluorine compound³⁾ | 1.0 |
| 10. Titanium oxide treated with a fluorine compound³⁾ | 5.0 |
| 11. Iron oxide red treated with a fluorine compound³⁾ | 0.9 |
| 12. Iron oxide yellow treated with a fluorine compound³⁾ | 2.0 |
| 13. Iron oxide black treated with a fluorine compound³⁾ | 1.0 |
| 14. Ethyl alcohol | 15.0 |
| 15. Glycerin | 3.0 |
| 16. Magnesium sulfate | 1.0 |
| 17. Antiseptics | q.s. |
| 18. Perfume | Balance |
| 19. Purified water | 25.1 |

| | |
|---|---|
| 1) FL-5 from Shin-Etsu Chemical Co., Ltd. 2)KMP-590 from Shin-Etsu Chemical Co., Ltd. 3) Coated with 5% of perfluoroalkylethyl phosphoric acid diethanolamine salt. | |

### Preparation procedures

A: Components 7 to 13 were mixed.
B: Components 1 to 6 were mixed at 70 °C, which was added to the mixture prepared in the step A and dispersed.
C: Components 14 to 17 and 19 were heated at 40 °C and then added to the mixture prepared in the step B and emulsified. The emulsion was then cooled to which Component 18 was added.

The liquid foundation thus obtained was non-tacky. It spread smoothly and lasted long on the skin to give very refreshing feel. It was confirmed to be stable with time and temperature.

### Example 26: Lip cream

| Components | % |
|---|---|
| 1. Decamethylcyclopentasiloxane | 40.0 |
| 2. Isoparaffin (b.p. 155 °C) | 10.0 |
| 3. Squalane | 10.0 |
| 4. Lanolin | 2.0 |
| 5. Trimethylsiloxysilicate | 3.0 |
| 6. Microcrystalline wax | 3.0 |
| 7. Silicone (Example VII) | 3.0 |
| 8. Lauroyl glutamate dibutyl amide | 5.0 |
| 9. Sodium lactate | 0.3 |
| 10. Sodium L-Glutamate | 0.3 |
| 11. Sodium hyaluronate | 0.1 |
| 12. Sorbitol | 0.5 |
| 13. Glycerin | 5.0 |
| 14. Red No. 202 | q.s. |
| 15. Menthol | q.s. |
| 16. Antiseptics | q.s. |
| 17. Perfume | q.s. |
| 18. Purified water | Balance |

### Preparation procedures

A: Components 1 to 8 were heat-mixed.
B: Components 9 to 16 were dissolved in Component 18 by heating.
C: The solution prepared in the step B was slowly added to the mixture prepared in the step A and emulsified while stirring. To the emulsion obtained, Component 17 was added, which was put in a capsule.

The solid water-in-oil type lip cream thus obtained was non-tacky and non-greasy. It spread smoothly on the lips to give moisturized and refreshing feel. It was confirmed to be stable with time.

### Example 27: Milky lotion

| Components | % |
|---|---|
| 1. Decamethylcyclopentasiloxane | 18.0 |
| 2. Dimethylpolysiloxane (6 mm²/sec) | 6.0 |
| 3. Squalane | 5.0 |
| 4. Neopentyl glycol dioctanoate | 3.0 |
| 5. α-monooleyl glycerylether | 1.0 |
| 6. Silicone (Example V) | 2.0 |
| 7. Aluminum distearate | 0.2 |
| 8. Magnesium sulfate | 0.7 |
| 9. Glycerin | 5.0 |
| 10. Antiseptics | q.s. |
| 11. Perfume | q.s. |
| 12. Purified water | Balance |

### Preparation procedures

A: Components 1 to 7 were heat-mixed.
B: Components 8 to 10 were dissolved in Component 12 by heating.
C: The solution prepared in the step B was slowly added to the mixture prepared in the step A and emulsified while stirring. The emulsion obtained was cooled, to which Component 11 was added.

The milky lotion thus obtained had fine texture. It was non-tacky and non-greasy. It spread smoothly on the skin to give moisturized and refreshing feel. It was confirmed to be stable with time.

### Example 28: Sun screen cream

| Components | % |
|---|---|
| 1. Decamethylcyclopentasiloxane | 18.0 |
| 2. Methylphenylpolysiloxane | 2.0 |
| 3. Liquid paraffin | 1.5 |
| 4. Silicone (Example V) | 4.0 |
| 5. Octyl paramethoxycinnamate | 5.0 |
| 6. 1,3-butylene glycol | 4.0 |
| 7. Sodium chloride | 1.0 |
| 8. Antiseptics | q.s. |
| 9. Perfume | q.s. |
| 10. Purified water | Balance |

### Preparation procedures

A: Components 1 to 5 were mixed.
B: Components 6 to 8 and 10 were mixed.
C: The mixture prepared in the step B was slowly added to the mixture prepared in the step A and emulsified while stirring. The emulsion obtained was cooled, to which Component 9 was added.

The sun screen cream thus obtained had fine texture. It was non-tacky and non-greasy. It spread smoothly on the skin to give moisturized and refreshing feel. The applied cream was resistant to water and sweat and lasted long, keeping ultraviolet light protective effect. It was confirmed to be stable with time.

### Example 29:Deodorant

| Components | % |
|---|---|
| 1. Decamethylcyclopentasiloxane | 12.0 |
| 2. Dimethylpolysiloxane (6 mm²/sec) | 4.0 |
| 3. Silicone (Example III) | 1.0 |
| 4. Propylene glycol | 31.0 |
| 5. Triclosan | 0.1 |
| 6. Glycerin | 15.0 |
| 7. Antiseptics | q.s. |
| 8. Perfume | q.s. |
| 9. Purified water | Balance |

### Preparation procedures

A: Components 1 to 3 were mixed.
B: Component 5 was dissolved in Component 4, to which Components 6 to 9 were added and mixed.
C: The mixture prepared in the step B was added to the mixture prepared in the step A and emulsified while stirring vigorously.
D: In a aerosol can, 65 g of the emulsion obtained in the step C, 35 g of propellant, i.e., a mixture of n-butane, isobutene and propane, were placed.

The deodorant thus obtained was non-tacky and non-greasy. It did not drip off even when it was oversprayed. The deodorant effect lasted long. It was confirmed to be stable with time.

### Example 30: Wash-off type pack

| Components | % |
|---|---|
| 1. Dimethylpolysiloxane (6 mm²/sec) | 3.0 |
| 2. Silicone (Example VI) | 2.0 |
| 3. Kaolin | 30.0 |
| 4. Carboxyvinyl polymer | 0.4 |
| 5. 1,3-butylene glycol | 10.0 |
| 6. Glycerin | 20.0 |
| 7. Antiseptics | q.s. |
| 8. Perfume | q.s. |
| 9. Purified water | Balance |

### Preparation procedures

A: Components 1, 2 and 8 were mixed.
B: Components 4 to 7 and 9 were mixed, to which Component 3 was added and mixed by stirring.
C: The mixture prepared in the step A was added to the mixture prepared in the step B and emulsified.

The wash-off type pack thus obtained spread lightly on the skin. It had high cleaning effect. After the pack was washed away, the skin was moisturized and silky smooth without tackiness. It was confirmed to be stable with time.

### Example 31: Roll-on type antiperspirant

| Components | % |
|---|---|
| 1. Crosslinked polyether-modified silicone¹⁾ | 20.0 |
| 2. Dimethylpolysiloxane (6 mm²/sec) | 10.0 |
| 3. Crosslinked dimethylpolysiloxane²⁾ | 14.3 |
| 4. Decamethylcyclopentasiloxane | 30.0 |
| 5. Silicone (Example I) | 0.5 |
| 6. Bentonite modified with an organic compound | 0.2 |
| 7. Aluminum zirconium tetrachlorohydrate | 20.0 |
| 8. Zinc oxide treated with dimethylhydrogensiloxane | 5.0 |
| 9. Perfume | q.s. |

| | |
|---|---|
| 1) KSG-21 from Shin-Etsu Chemical Co., Ltd. 2) KSG-15 from Shin-Etsu Chemical Co., Ltd. | |

### Preparation procedures

A: Components 1 to 6 were mixed.
B: To the mixture prepared in the step A, Components 7 to 9 were added and dispersed.

The roll-on type antiperspirant thus obtained spread lightly on the skin to give refreshing feel without tackiness and greasiness. It was confirmed to be stable with time.

### Example 32: Antiperspirant

| Components | % |
|---|---|
| 1. Decamethylcyclopentasiloxane | 30.0 |
| 2. Silicone (Example V) | 1.0 |
| 3. Polyoxyethylenesorbitan monooleate(20E.O.) | 0.5 |
| 4. Aluminum zirconium tetrachlorohydrate glycine salt | 20.0 |
| 5. Purified water | 48.5 |

### Preparation procedures

A: Components 1 and 2 were mixed.
B: Component 4 was dissolved in Component 5, to which Component 3 was added.
C: To the mixture prepared in the step A, the mixture prepared in the step B was slowly added and emulsified while stirring.

The antiperspirant thus obtained spread lightly on the skin to give refreshing feel without tackiness and greasiness. Moreover, it did not whiten the skin very much. It was confirmed to be stable with temperature and time.

### Example 33: Eyeliner

| Components | % |
|---|---|
| 1. Methyl tristrimethylsiloxy silane¹⁾ | 14.0 |
| 2. Silicone (Example II) | 3.0 |
| 3. Organosilicone resin²⁾ | 15.0 |
| 4. Montmorillonite modified with dioctadecyldimethylammonium | 3.0 |
| 5. Dispersion of iron oxide black³⁾ | 25.0 |
| 6. 1,3-butylene glycol | 5.0 |
| 7. Sodium dehydro acetate | q.s. |
| 8.Antiseptics | q.s. |
| 9. Purified water | Balance |

| | |
|---|---|
| 1) TMF-1.5 from Shin-Etsu Chemical Co., Ltd. 2) KF-7312T from Shin-Etsu Chemical Co., Ltd. 3) The Dispersion was prepared by dispersing 40 mass% of iron oxide black in 10 mass% of KP-574 from Shin-Etsu Chemical Co., Ltd, and 50 mass% of decamethylcyclopentasiloxane. | |

### Preparation procedures

A: Components 1 to 4 were mixed, to which Component 5 was added and dispersed.
B: Components 6 to 9 were mixed.
C: To the dispersion prepared in the step A, the mixture prepared in the step B was slowly added and emulsified.

The eyeliner thus obtained spread lightly and easy to draw on the eyelids to give refreshing feel without tackiness. The applied eye liner was resistant to water and sweat and lasted long. It was confirmed to be stable with time.

### Example 34: Cream eye shadow

| Components | % |
|---|---|
| 1. Decamethylcyclopentasiloxane | 15.0 |
| 2. Dimethylpolysiloxane (6 mm²/sec) | 10.0 |
| 3. Silicone (Example V) | 2.0 |
| 4. PEG(10) lauryl ether | 0.5 |
| 5. Silicone-treated chromium oxide¹⁾ | 6.2 |
| 6. Silicone-treated ultramarine blue¹⁾ | 4.0 |
| 7. Titanium-coated mica treated with silicone ¹⁾ | 6.0 |
| 8. Sodium chloride | 2.0 |
| 9. Propylene glycol | 8.0 |
| 10. Antiseptics | q.s. |
| 11. Perfume | q.s. |
| 12. Purified water | Balance |

| | |
|---|---|
| 1) Ninety-seven grams of powder was treated with 3 g of KF-9909, ex Shin-Etsu Chemical Co., Ltd., dissolved in isopropyl alcohol and then isopropyl alcohol was distilled off. | |

### Preparation procedures

A: Components 1 to 4 were mixed, to which Components 5 to 7 were added and dispersed.
B: Components 8 to 10 were dissolved in Component 12.
C: To the mixture prepared in the step A, the solution prepared in the step B was slowly added and emulsified while stirring. To the emulsion thus obtained, Component 11 was added.

The cream eye shadow thus obtained spread lightly on the eyelids. It gave refreshing feel without greasiness and powderiness. The applied eye shadow was resistant to water and sweat and lasted long. It was confirmed to be stable with temperature and time.

### Example 35: Eyeliner

| Components | % |
|---|---|
| 1. Decamethylcyclopentasiloxane | 6.0 |
| 2. Dimethylpolysiloxane (6 mm²/sec) | 5.0 |
| 3. Jojoba oil | 2.0 |
| 4. Silicone (Example II) | 1.0 |
| 5. Alkyl/polyether co-modified silicone¹⁾ | 1.0 |
| 6. Acrylic silicone resin²⁾ | 15.0 |
| 7. Silicone-treated iron oxide black³⁾ | 20.0 |
| 8. Ethyl alcohol | 5.0 |
| 9. Antiseptics | q.s. |
| 10. Purified water | Balance |

| | |
|---|---|
| 1) KF-6038 from Shin-Etsu Chemical Co., Ltd. 2) KP-545 from Shin-Etsu Chemical Co., Ltd. 3) Ninety-eight grams of iron oxide black powder was treated with 2 g of KF-9909, ex Shin-Etsu Chemical Co., Ltd., dissolved in isopropyl alcohol and then isopropyl alcohol was distilled off. | |

### Preparation procedures

A: Components 1 to 6 were mixed, to which Component 7 was added and dispersed.
B: Components 8 and 9 were dissolved in Component 10 by heating.
C: To the mixture prepared in the step A, the mixture prepared in the step B was slowly added and emulsified while stirring.

The eyeliner thus obtained spread lightly on the eyelids. It gave refreshing and moisturized feel without greasiness and powderiness. The applied eyeliner was water repellant, resistant to water and sweat, and lasted long. It was confirmed to be stable with temperature and time.

### Example 36: W/O hand cream

| Components | % |
|---|---|
| 1. Decamethylcyclopentasiloxane | 30.0 |
| 2. Liquid paraffin | 10.0 |
| 3. Amino-modified silicone gum¹⁾ | 15.0 |
| 4. Silicone (Example VII) | 4.0 |
| 5. Distearyldimethylammonium chloride | 0.8 |
| 6. Vitamin E acetate | 0.1 |
| 7. Polyethylene glycol 4000 | 1.0 |
| 8. Glycerin | 10.0 |
| 9. Smectone | 1.2 |
| 10. Antiseptics | q.s. |
| 11. Perfume | q.s. |
| 12. Purified water | Balance |

| | |
|---|---|
| 1) Amine equivalent of 70,000g/mol | |

### Preparation procedures

A: Components 1, 3 were heat-mixed, to which Components 2, 4, 5, 6 and 10 were added while heating.
B: Components 7 to 9 and 12 were heat-mixed.
C: To the mixture prepared in step A, the mixture prepared in the step B was slowly added and emulsified. The emulsion was then cooled, to which Component 11 was added.

The hand cream thus obtained was non-tacky and spread lightly on the hands to give moisturized feel. It effectively protected the hands from water. It was confirmed to be stable with time.

### Example 37: W/O/W type cream

| Components | % |
|---|---|
| 1. Crosslinked polyether-modified silicone¹⁾ | 4.3 |
| 2. Cetyl isooctanoate | 5.0 |
| 3. Crosslinked alkyl-modified silicone²⁾ | 1.0 |
| 4. Silicone (Example II) | 0.7 |
| 5. Decamethylcyclopentasiloxane | 5.0 |
| 6. Methyl glucoside dioleate | 1.5 |
| 7. Isohexadecane | 3.5 |
| 8. Magnesium sulfate | 0.5 |
| 9. Propylene glycol | 5.0 |
| 10. Purified water | 39.5 |
| 11. Cetyl alcohol | 1.0 |
| 12. PEG-10 soya seterol | 2.0 |
| 13. Antiseptics | q.s. |
| 14. Perfume | q.s. |
| 15. Purified water | 31.0 |

| | |
|---|---|
| 1) KSG-210 from Shin-Etsu Chemical Co., Ltd. 2) KSG-44 from Shin-Etsu Chemical Co., Ltd. | |

### Preparation procedures

A: Components 8 to 10 were mixed.
B: Components 1 to 7 were mixed, which then was added to the mixture prepared in the step A.
C: Components 11 to 13 and 15 were mixed, to which the mixture prepared in the step B was added and emulsified while stirring.
D: To the emulsion prepared in the step C, Component 14 was added.

The cream thus obtained spread lightly on the skin to give refreshing feel without tackiness and greasiness. It gave a translucent finish which lasted long. It was confirmed to be stable with time and temperature.

### Example 38: Non-aqueous emulsion

| Components | % |
|---|---|
| 1. Crosslinked dimethylpolysiloxane¹⁾ | 30.0 |
| 2. Decamethylcyclopentasiloxane | 15.0 |
| 3. Dimethylpolysiloxane (6 mm²/sec) | 6.0 |
| 4. Crosslinked alkyl-modified silicone compound²⁾ | 3.0 |
| 5. Silicone (Example V) | 1.0 |
| 6. Dimethyl distearyl ammonium hectorite | 2.0 |
| 7. 1,3-butylene glycol | 43.0 |

| | |
|---|---|
| 1) KSG-15 from Shin-Etsu Chemical Co., Ltd. 2) KSG-43 from Shin-Etsu Chemical Co., Ltd. | |

### Preparation procedures

A: Components 1 to 6 were mixed.
B: Component 7 was added to the mixture prepared in the step A and emulsified.

The nonaqueous emulsion thus obtained spread lightly on the skin to give softened feel without tackiness and greasiness. It was confirmed to be stable.

### Example 39: O/W type sunscreen milky lotion

| Components | % |
|---|---|
| 1. Acrylic silicone resign¹⁾ | 10.0 |
| 2. Dispersion of titanium oxide²⁾ | 15.0 |
| 3. Dispersion of zinc oxide³⁾ | 15.0 |
| 4. Decamethylcyclopentasiloxane | 16.0 |
| 5. Neopentylglycol dioctanoate | 8.0 |
| 6. Polymethylsilsesquioxane | 3.0 |
| 7. Polyether-modified silicone⁴⁾ | 4.0 |
| 8. Polyether-modified silicone⁵⁾ | 1.5 |
| 9. Polyoxyethylene hydrogenated castor oil | 1.5 |
| 10. 1,3-butylene glycol | 5.0 |
| 11. Purified water | 20.5 |

| | |
|---|---|
| 1) KP-545 from Shin-Etsu Chemical Co., Ltd. 2) Forty grams of titanium oxide, MT-100TV, ex Tayca Co., were dispersed in 10 g of the silicone of Example V dissolved in 50 g of decamethylcyclopentasiloxane. 3) Fifty grams of zinc oxide, MZ505S, ex Tayca Co., were dispersed in 8 g of silicone of Example VII dissolved in 42 g of decamethylcyclopentasiloxane. 4) KF-6011P from Shin-Etsu Chemical Co., Ltd. 5) KF-6013P from Shin-Etsu Chemical Co., Ltd. | |

### Preparation procedures

A: Components 7 to 11 were mixed.
B: Components 1 to 6 were mixed and dispersed, which was then added to the mixture prepared in the step A and emulsified.

The sunscreen milky lotion thus obtained had fine texture. It spread lightly on the skin to give moisturized feel without tackiness. The applied lotion stayed long, maintaining ultraviolet light protective effect. It was confirmed to be stable with time and temperature.

### Example 40: O/W/O type milky lotion

| Components | % |
|---|---|
| 1. Crosslinked alkyl/polyglycerin-modified | |
| silicone¹⁾ | 3.0 |
| 2. Silicone (Example II) | 1.0 |
| 3. Glyceryl triisooctanoate | 14.0 |
| 4. Crosslinked alkyl-modified silicone compoud²⁾ | 5.0 |
| 5. Sucrose stearate | 3.0 |
| 6. Glycerin | 5.0 |
| 7. 1,3-butylene glycol | 5.0 |
| 8. Antiseptics | q.s. |
| 9. Purified water | 60.0 |
| 10. Macadamia nut oil | 2.0 |
| 11. Cetyl alcohol | 2.0 |
| 12. Perfume | q.s. |

| | |
|---|---|
| 1) KSG-810 from Shin-Etsu Chemical Co., Ltd. 2) KSG-41 from Shin-Etsu Chemical Co., Ltd. | |

### Preparation procedures

A: Components 1 to 4 were mixed.
B: Components 5 and 9 were heat-mixed.
C: Components 10 to 12 were heat-mixed.
D: The mixture prepared in the step C was added to the mixture prepared in the step B and emulsified while stirring. The emulsion was then cooled.
E: The emulsion obtained in the step D was added to the mixture obtained in the step A and emulsified while heating.

The milky lotion thus obtained spread lightly on the skin to give refreshing feel without tackiness and greasiness. The applied lotion was translucent and stayed long. It was confirmed to be stable with time and temperature.

### Example 41: Powder foundation

| Components | % |
|---|---|
| 1. Vaseline | 2.5 |
| 2. Squalane | 3.0 |
| 3. Crosslinked alkyl-modified silicone¹⁾ | 0.5 |
| 4. Glyceryl trioctanoate | 2.0 |
| 5. Silicone-treated mica²⁾ | 40.0 |
| 6. Silicone-treated talc²⁾ | Balance |
| 7. Silicone-treated titanium oxide³⁾ | 10.0 |
| 8. Silicone-treated titanium oxide fine particles³⁾ | 5.0 |
| 9. Silicone-treated barium sulfate²⁾ | 10.0 |
| 10. Pigment | q.s. |
| 11. Composite powder of phenyl-modified hybrid silicone ⁴⁾ | 2.0 |
| 12. Silicone powder⁵⁾ | 2.5 |
| 13. Antiseptics | q.s. |
| 14. Perfume | q.s. |

| | |
|---|---|
| 1) KSG-44 from Shin-Etsu Chemical Co., Ltd. 2) Ninety-seven g of powder was treated with 3 g of silicone prepared in Example V dissolved in isopropyl alcohol and then isopropyl alcohol was distilled off. 3) Ninety-eight grams of powder was treated with 2 g of KF-9909, ex Shin-Etsu Chemical Co., Ltd., dissolved in isopropyl alcohol and then isopropyl alcohol was distilled off. 4) KSP-300 from Shin-Etsu Chemical Co., Ltd. 5) KMP-590 from Shin-Etsu Chemical Co., Ltd. | |

### Preparation procedures

A: Components 4 to 13 were mixed.
B: Components 1 to 3 were mixed and added to the mixture prepared in the step A.
C: To the homogenous mixture prepared in the step B, Component 14 was added. The mixture obtained was press-molded into a powder foundation in a metal mold.

The powder foundation thus obtained was non-tacky and spread lightly on the skin. It had high affinity for the skin to give a gloss finish which lasted long on the skin.

### Example 42: Cream foundation

| Components | % |
|---|---|
| 1. Crosslinked polyether-modified silicone¹⁾ | 3.0 |
| 2. Crosslinked alkyl-modified silicone²⁾ | 1.5 |
| 3. Silicone (Example V) | 1.0 |
| 4. Glyceryl trioctanoate | 4.0 |
| 5. Dimethylpolysiloxane (6 mm²/sec) | 5.0 |
| 6. Decamethylcyclopentasiloxane | 5.0 |
| 7. Composite of fluorine-modified hybrid silicone³⁾ | 2.5 |
| 8. Silicone-treated pigment⁴⁾ | 8.0 |
| 9. Acrylic silicone resin⁵⁾ | 5.0 |
| 10. Dipropylene glycol | 5.0 |
| 11. Sodium citrate | 0.2 |
| 12. Antiseptics | q.s. |
| 13. Perfume | q.s. |
| 14. Purified water | 59.3 |

| | |
|---|---|
| 1) KSG-210 from Shin-Etsu Chemical Co., Ltd. 2) KSG-43 from Shin-Etsu Chemical Co., Ltd. 3) KSP-200 from Shin-Etsu Chemical Co., Ltd. 4) Ninety-eight grams of the pigment was treated with 2 g of KF-9909, ex Shin-Etsu Chemical Co., Ltd., dissolved in isopropyl alcohol and then isopropyl alcohol was distilled off. 5) KP-545 from Shin-Etsu Chemical Co., Ltd. | |

### Preparation procedures

A: Components 1 to 7 were mixed.
B: Components 10 to 13 were dissolved in Component 14, which was then added to the mixture prepared in the step A.
C: Components 8 and 9 were mixed and added to the mixture prepared in the step B.

The cream foundation thus obtained was non-tacky and spread lightly on the skin. It had high affinity for the skin to give a matt finish which lasted long.

### Example 43: W/O type compact foundation

| Components | % |
|---|---|
| 1. Ceresin | 5.5 |
| 2. Microcrystalline wax | 1.0 |
| 3. Liquid paraffin | 3.0 |
| 4. Crosslinked alkyl-modified silicone¹⁾ | 1.0 |
| 5. Polypropylene glycol dicaprinate | 3.0 |
| 6. Silicone (Example V) | 1.0 |
| 7. Crosslinked alkyl/polyether-modified silicone²⁾ | 8.0 |
| 8. Dimethylpolysiloxane (6 mm²/sec) | 15.5 |
| 9. Titanium oxide treated with oil | 10.0 |
| 10. Pigment treated with a compound having an | |
| alkyl group³⁾ | q.s. |
| 11. Lecithin | 0.3 |
| 12. Polyoxyethylene sorbitan monooleate | 0.5 |
| 13. Dipropylene glycol | 8.0 |
| 14. Sodium citrate | 0.2 |
| 15. Purified water | Balance |

| | |
|---|---|
| 1) KSG-41 from Shin-Etsu Chemical Co., Ltd. 2) KSG-330 from Shin-Etsu Chemical Co., Ltd. 3) Ninety-eight grams of pigment was treated with 2 g of AES-3083, ex Shin-Etsu Chemical Co., Ltd., dissolved in isopropyl alcohol and then isopropyl alcohol was distilled off. | |

### Preparation procedures

A: Components 1 to 8 were mixed.
B: Components 9 to 13 were mixed.
C: Components 14 and 15 were mixed, which was then added to the mixture prepared in the step B and heated.
D: The mixture prepared in the step C was added to the mixture prepared in the step A and emulsified.

The compact foundation thus obtained was non-tacky and non-greasy in spite of its relatively high content of unctuous agents. It spread lightly on the skin to give refreshing feel. The applied foundation lasted long with high affinity for the skin.

### INDUSTRIAL APPLICABILITY

As described in detail above, the glycerin-modified silicone of the present invention is highly hydrophilic, and easy to prepare. The cosmetic of the present invention comprising the glycerin-modified silicone gives moisturized and non-tacky feel to the touch without causing twitchiness to the skin. The cosmetic is excellently stable, too.

## Claims

1. A glycerin-modified silicone represented by the following formula (1):
R¹ₐR²_{b}R³_{c}SiO_{(4-a-b-c)/2} (1)
wherein R¹ is, independently, an unsubstituted or a partially halogen-substituted alkyl, aryl, aralkyl, aminoalkyl, or carboxyalkyl group, of 1 to 30 carbon atoms,
R² is, independently, an organosiloxyalkyl group represented by the following formula (3): 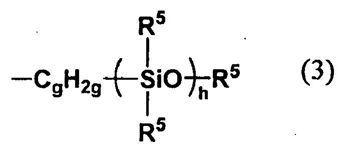 wherein R⁵ is an unsubstituted or a partially halogen-substituted alkyl group of 1 to 30 carbon atoms, g is a number of from 1 to 5, and h is a number of from 1 to 500,
R³ is a group represented by the following formula (4): 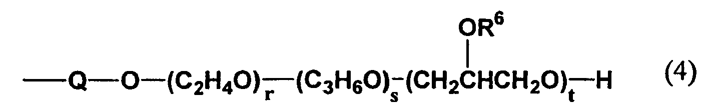 wherein Q is a C2-20 divalent group, r is a number of from 1 to 200, s is a number of from 0 to 200, R⁶ is a hydrogen atom, a C1-30 alkyl group or an organic group of the formula: -(CO)-R⁷, wherein R⁷ is a C1-30 hydrocarbon group, and t is a number of from 1 to 10,
a is a number of from 0.5 to 2.5, b is a number of from 0 to 1.5, and c is a number of from 0.001 to 1.5.

2. The glycerin-modified silicone according to claim 1, wherein t ranges from 1 to 3.

3. The glycerin-modified silicone according to claim 1 or 2, wherein r ranges from 1 to 5 and s is 0.

4. The glycerin-modified silicone according to any one of claims 1 to 3, wherein R¹ is a methyl group.

5. A cosmetic comprising the glycerin-modified silicone according to any one of claims 1 to 4.

6. The cosmetic according to claim 5, wherein the cosmetic is in the form of emulsion, and comprises the glycerin-modified silicone in an amount of from 0.01 to 20 mass % based on total mass of the cosmetic.

7. The cosmetic according to claim 5, wherein the cosmetic is a cleansing cosmetic, and comprises the glycerin-modified silicone in an amount of from 0.01 to 40 mass%.

## Patentansprüche

1. Glycerinmodifiziertes Silikon der folgenden Formel (1) :
R¹ₐR²_{b}R³_{c}SiO_{(4-a-b-c)/2} (1)
worin R¹ unabhängig für eine unsubstituierte oder teilweise halogensubstituierte Alkyl-, Aryl-, Aralkyl-, Aminoalkyl- oder Carboxyalkylgruppe mit 1 bis 30 Kohlenstoffatomen steht,
R² unabhängig für eine Organosiloxyalkylgruppe der folgenden Formel (3) steht: 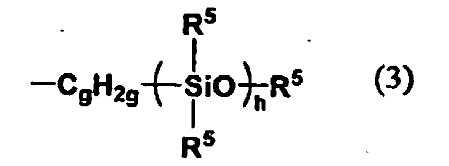 worin R⁵ für eine unsubstituierte oder teilweise halogensubstituierte Alkylgruppe mit 1 bis 30 Kohlenstoffatomen steht, g für eine Zahl von 1 bis 5 steht und h für eine Zahl von 1 bis 500 steht, R³ für eine Gruppe der folgenden Formel (4) steht: 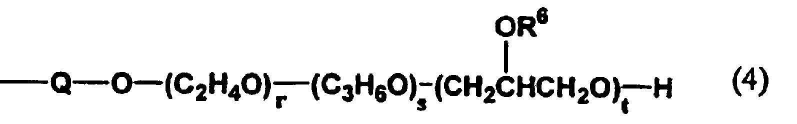 worin Q für eine zweiwertige C2-C20-Gruppe steht, r für eine Zahl von 1 bis 200 steht, s für eine Zahl von 0 bis 200 steht, R⁶ für ein Wasserstoffatom, eine C1-30-Alkylgruppe oder eine organische Gruppe der Formel - (CO) -R⁷, worin R⁷ eine C1-C30-Kohlenwasserstoffgruppe bedeutet, steht und t für eine Zahl von 1 bis 10 steht,
a für eine Zahl von 0,5 bis 2,5 steht, b für eine Zahl von 0 bis 1,5 steht und c für eine Zahl von 0,001 bis 1,5 steht.

2. Glycerinmodifiziertes Silikon nach Anspruch 1, wobei t im Bereich von 1 bis 3 liegt.

3. Glycerinmodifiziertes Silikon nach Anspruch 1 oder 2, wobei r im Bereich von 1 bis 5 liegt und s für 0 steht.

4. Glycerinmodifiziertes Silikon nach einem der Ansprüche 1 bis 3, wobei R¹ für eine Methylgruppe steht.

5. Kosmetikum, umfassend das glycerinmodifizierte Silikon nach einem der Ansprüche 1 bis 4.

6. Kosmetikum nach Anspruch 5, wobei das Kosmetikum in Form einer Emulsion vorliegt und das glycerinmodifizierte Silikon in einer Menge von 0,01 bis 20 Masse-%, bezogen auf die Gesamtmasse des Kosmetikums, umfasst.

7. Kosmetikum nach Anspruch 5, wobei das Kosmetikum ein reinigendes Kosemtikum ist und das glycerinmodifizierte Silikon in einer Menge von 0,01 bis 40 Masse-% umfasst.

## Revendications

1. Silicone modifié par le glycérol représenté par la formule (1) suivants :
R¹ₐR²_{b}R³_{c}SiO_{(4-a-b-c)/2} (1)
dans laquelle
R¹ est, indépendamment, un groupe alkyle, aryle, aralkyle, aminoalkyle ou carboxyalkyle, de 1 à 30 atomes de carbone, non substitué ou partiellement substitué par des atomes d'halogène,
R² est, indépendamment, un groupe organosiloxyalkyle représenté par la formule (3) suivante : 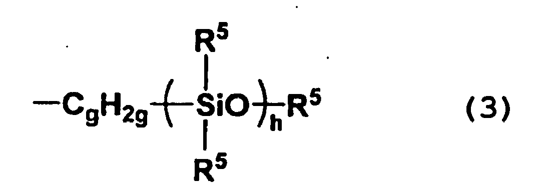 dans laquelle
R⁵ est un groupe alkyle, de 1 à 30 atomes decarbone, non substitué ou partiellement substitué par des atomes d'halogène,
g est un nombre de 1 à 5 et
h est un nombre de 1 à 500,
R³ est un groupe représenté par la formule (4) suivante : 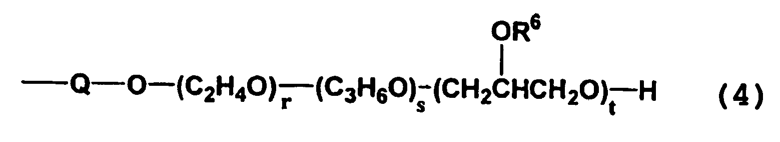
dans laquelle
Q est un groupe divalent en C2-20,
r est un nombre de 1 à 200,
s est un nombre de 0 à 200,
R⁶ est un atome d'hydrogène, un groupe alkyle en C1-30 ou un groupe organique représenté par la formule :
- (CO) -R⁷,
dans laquelle
R⁷ est un groupe hydrocarboné en C1-30 et
t est un nombre de 1 à 10,
a est un nombre de 0,5 à 2,5,
b est un nombre de 0 à 1,5 et
c est un nombre de 0,001 à 1,5.

2. Silicone modifié par le glycérol selon la revendication 1, dans lequel t va de 1 à 3.

3. Silicone modifié par le glycérol selon la revendication 1 ou 2, dans lequel r va de 1 à 5 et s vaut 0.

4. Silicone modifié par le glycérol selon l'une quelconque des revendications 1 à 3, dans lequel R¹ est un groupe méthyle.

5. Produit cosmétique comprenant le silicone modifié par le glycérol selon l'une quelconque des revendications 1 à 4.

6. Produit cosmétique selon la revendication 5, le produit cosmétique étant sous la forme d'une émulsion et comprenant le silicone modifié par le glycérol en une quantité de 0,01 à 20 % en masse sur la base de la masse totale du produit cosmétique.

7. Produit cosmétique selon la revendication 5, le produit cosmétique étant un produit cosmétique nettoyant et comprenant le silicone modifié par le glycérol en une quantité de 0,01 à 40 % en masse.
